(19) Europäisches Patentamt
European Patent Office
Office européen des brevets

(11) **EP 2 227 539 B1**

(12) **FASCICULE DE BREVET EUROPEEN**

(45) Date de publication et mention de la délivrance du brevet:
**09.08.2017 Bulletin 2017/32**

(21) Numéro de dépôt: **08872538.7**

(22) Date de dépôt: **12.12.2008**

(51) Int Cl.:
***C12N 1/18*** *(2006.01)*     ***A61K 36/064*** *(2006.01)*

(86) Numéro de dépôt international:
**PCT/FR2008/001729**

(87) Numéro de publication internationale:
**WO 2009/103884 (27.08.2009 Gazette 2009/35)**

(54) **COMPOSITION POUR L'ALIMENTATION HUMAINE ET/OU ANIMALE, SES UTILISATIONS, LEVURES**

ZUSAMMENSETZUNG FÜR EINE MENSCHEN- UND/ODER TIERNAHRUNG, ANWENDUNGEN DAVON UND HEFEN DAMIT

COMPOSITION FOR HUMAN AND/OR ANIMAL NUTRITION, USES THEREOF AND YEASTS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **26.12.2007  FR 0760377**
**12.03.2008  FR 0801342**

(43) Date de publication de la demande:
**15.09.2010  Bulletin 2010/37**

(83) **Déclaration conformément à la règle 32(1) CBE (solution de l'expert)**

(73) Titulaires:
• **Lesaffre et Compagnie**
**75001 Paris (FR)**
• **Université d'Auvergne Clermont 1**
**63001 Clermont-Ferrand Cedex 1 (FR)**
• **Université de Droit et de la Santé de Lille 2**
**59800 Lille (FR)**

(72) Inventeurs:
• **SIMON, Jean-Luc**
**F-59000 Lille (FR)**
• **PIGNEDE, Georges**
**F-59700 Marcq en Baroeul (FR)**
• **VANDEKERCKOVE, Pascal**
**F-59650 Villeneuve d'Ascq (FR)**
• **POULAIN, Daniel**
**F-59242 Templeuve (FR)**
• **DESREUMAUX, Pierre**
**F-59700 Marq en Baroeul (FR)**

• **DARFEUILLE - MICHAUD, Arlette**
**F-63670 La Roche Blanche (FR)**
• **SIVIGNON, Adeline**
**F-63000 Clermont Ferrand (FR)**

(74) Mandataire: **Cabinet Plasseraud**
**66 rue de la Chaussée d'Antin**
**75440 Paris Cedex 09 (FR)**

(56) Documents cités:
**EP-A- 1 481 682     EP-A- 1 693 064**
**WO-A-2006/021965**

• **DALMASSO G. ET AL.: "Saccharomyces boulardii Inhibits Inflammatory Bowel Disease by Trapping T Cells in Mesenteric Lymph Nodes" GASTROENTEROLOGY, ELSEVIER, PHILADELPHIA, PA, vol. 131, no. 6, 22 décembre 2006 (2006-12-22), pages 1812-1825, XP005750982 ISSN: 0016-5085**
• **EDWARDS-INGRAM L. ET AL.: "Genotypic and physiological characterization of Saccharomyces boulardii, the probiotic strain of Saccharomyces cerevisiaev" APPLIED AND ENVIRONMENTAL MICROBIOLOGY, vol. 73, no. 8, avril 2007 (2007-04), pages 2458-2467, XP002484204 ISSN: 0099-2240**
• **CZERUCKA D. ET AL.: "Yeast as probiotics - Saccharomyces boulardii" ALIMENTARY PHARMACOLOGY & THERAPEUTICS, vol. 26, no. 6, septembre 2007 (2007-09), pages 767-778, XP002484394 ISSN: 0269-2813**

**Description**

La présente invention se rapporte aux domaines de la nutrition et de la santé humaine et/ou animale.

Elle concerne plus particulièrement de nouvelles souches de levure et de nouvelles levures obtenues à partir des nouvelles souches. Ces levures sont notamment utiles pour le confort du tractus digestif et/ou pour la prévention et/ou le traitement des désordres du tractus digestif humain ou animal.

De nombreux microorganismes ont déjà été décrits dans la littérature pour leurs applications bénéfiques chez l'homme sur le tractus digestif et pour leur intérêt nutritionnel, comme décrit, par exemple, dans WO 2006/021965.

Ces microorganismes sont communément désignés alors par le terme probiotiques qui correspond à des microorganismes vivants capables d'apporter à l'hôte un bénéfice sur la santé lorsqu'ils sont administrés en quantité suffisante (Joint FAO/WHO Expert Cunsultation Probiotics in food, FAO Food and nutrition paper Nr85, ISBN 92-5-105513-0).

Les bénéfices résultant de l'administration par voie orale des microorganismes dépendent très largement de la souche de microorganisme utilisée, mais aussi de sa forme d'administration. Au sein d'une même espèce, selon les souches employées, les effets observés sont en effet très fluctuants, parfois bénéfiques, parfois négatifs ou neutres comme par exemple au sein de l'espèce *Escherichia coli* où on peut trouver à la fois des souches pathogènes (types entéro-toxinogènes ou entéro-hémorragiques par exemple) et des souches bénéfiques comme la souche Nissle 1917 (M. de Vrese ; P.R. Marteau. Probiotics and Prebiotics: Effects on Diarrhea. 2007, J. Nutr., 137(3 Suppl. 2), 8035-811S). Il est ainsi actuellement impossible de prédire pour une souche donnée si un bénéfice en terme de santé humaine peut être escompté de l'administration de cette souche, ni même de prévoir la nature de son éventuel bénéfice ou son intensité.

Un certain nombre de souches de microorganismes, notamment parmi les levures et les bactéries lactiques, ont déjà été identifiés pour certains effets bénéfiques sur le tractus gastro-intestinal. Néanmoins, l'obtention d'une action bénéfique complète sur le tractus gastro-intestinal nécessite souvent l'administration concomitante de plusieurs souches de nature différente (I. Goktepe ; V.K. Juneja ; M. Ahmedna (eds). Probiotics in Food Safety and Human Health. 2006, CRC Taylor & Francis, ISBN 1-57444-514-6).

En outre, il a été observé que bon nombre de microorganismes, en particulier les bactéries lactiques, ont une action pro-inflammatoire. Cet effet pro-inflammatoire peut s'avérer particulièrement néfaste et non souhaitable, par exemple dans les maladies auto-immunes ou de déficiences immunitaires.

Certaines fractions de levures et/ou dérivés de levures ont été décrits pour leurs effets bénéfiques sur le tractus digestif.

Ainsi, les mannoprotéines dérivés de levures ont été décrites pour leur effet inhibiteur de l'adhésion des pathogènes. De même les parois de levures ont été décrites pour leur effet fibre. Cependant, il existe de nombreuses souches de levures *Saccharomyces cerevisiae* et, elles ne présentent pas toutes des effets bénéfiques ou les mêmes effets.

En outre, selon les souches utilisées et les formes de levure administrées, les effets peuvent être également très variables.

Aussi il subsiste le besoin de pouvoir disposer de nouvelles souches de microorganismes pouvant exercer un effet bénéfique sur la santé, de manière préventive et /ou curative sur des pathologies ou dysfonctionnements précis ou non, ou sur l'état de santé général tant physique que psychique.

L'invention a donc pour objet une nouvelle souche *Saccharomyces cerevisiae* déposée auprès de la Collection Nationale de Cultures de Microorganismes sous le n° CNCM I-3856, et une nouvelle souche *Saccharomyces cerevisiae* var.boulardii déposée auprès de la Collection Nationale de Cultures de Microorganismes sous le n° CNCM I-3799.

Sont également décrites ici une levure *Saccharomyces cerevisiae* obtenue à partir de la souche déposée auprès de la Collection Nationale de Cultures de Microorganismes sous le n° CNCM I-3856, et une levure *Saccharomyces* var. boulardii obtenue à partir de la souche déposée auprès de la Collection Nationale de Cultures de Microorganismes sous le n° CNCM I-3799.

Un autre objet de l'invention est une composition comprenant la souche de levure *Saccharomyces cerevisiae* déposée auprès de la Collection Nationale de Cultures de Microorganismes sous le n° CNCM I-3856, la souche de levure *Saccharomyces* var. boulardii déposée auprès de la Collection Nationale de Cultures de Microorganismes sous le n° CNCM I-3799, ou un de leurs mélanges. Dans certains modes de réalisation, la composition comprend en outre au moins un dérivé de levure *Saccharomyces cerevisiae* ou *Saccharomyces* var. boulardii choisi parmi les extraits de levure, les dérivés de parois, les glucanes pariétaux, les mannoprotéines pariétales, les fractions lipidiques de levure, les fractions d'acides nucléiques de levure (ARN, ADN).

La composition selon l'invention présente les avantages suivants :

- une capacité, en particulier dans ses formes sèches, à résister et survivre au passage de la barrière gastrique, ce qui permet d'optimiser ses effets sur le tractus gastro-intestinal;
- une action anti-inflammatoire;

- une absence d'effet pro-inflammatoire ou un très faible effet;
- une capacité à réduire les douleurs intestinales, et enfin
- une capacité à empêcher et à réduire l'adhésion et la colonisation par des bactéries pathogènes et/ou à caractère invasif du tractus gastro-intestinal, en particulier de l'intestin grêle et du colon.

Une telle nouvelle composition, présentant cette combinaison de caractéristiques, n'a encore jamais été décrite ou identifiée.

Elle présente donc un intérêt exceptionnel.

Un autre objet de l'invention est une utilisation de la composition précédente pour la préparation d'un complément alimentaire et/ou d'un probiotique et/ou d'un alicament et/ou d'un nutraceutique et/ou d'ingrédients fonctionnels et/ou d'un cosméceutique, destinée à l'homme et/ou à l'animal.

L'invention se rapporte, par ailleurs, à une utilisation de la composition telle que définie précédemment pour la préparation de compositions alimentaires destinées à améliorer le confort gastro-intestinal et/ou améliorer la flore intestinale.

L'invention a également pour objet une utilisation de la composition telle que définie précédemment pour la préparation d'un médicament destiné au traitement et /ou à la prévention des désordres intestinaux, des troubles fonctionnels intestinaux ou des maladies gastro-intestinales.

Un objet de l'invention est une utilisation de la composition telle que définie précédemment pour la préparation d'un médicament destiné au traitement et/ou à la prévention des pathologies ou troubles de l'intestin signalés par un état d'hyperalgésie.

Enfin, un dernier objet de l'invention est un kit comprenant au moins une souche de levure telle que définie précédemment dans une forme adaptée à une administration orale. Dans certains modes de réalisation, le kit comprend en outre au moins un dérivé de levure tel que défini précédemment.

La souche, déposée par la Demanderesse sous le Traité de Budapest auprès de la Collection Nationale de Cultures de Microorganismes (Institut Pasteur Paris) sous le n°CNCM I-3856, sera appelée « ScPro1 » dans un but de concision.

La souche, déposée également par la Demanderesse sous le Traité de Budapest auprès de la Collection Nationale de Cultures de Microorganismes (Institut Pasteur Paris) sous le n°CNCM I-3799, sera dénommée « SCB1 » dans un but de concision.

Enfin, dans un dernier but de concision, le dérivé de levure *Saccharomyces cerevisiae* choisi parmi les extraits de levure, les dérivés de parois, les glucanes pariétaux, les mannoprotéines pariétales, les fractions lipidiques de levure, les fractions d'acides nucléiques de levure (ARN, ADN), et leurs mélanges, sera dénommé « dérivé ».

Par probiotique, on entend désigner des microorganismes vivants qui, lorsqu'ils sont intégrés en quantité suffisante, exercent un effet positif sur la santé, le confort et le bien-être au-delà des effets nutritionnels traditionnels.

Par alicament ou nutraceutique ou aliment fonctionnel ou cosméceutique, on entend un aliment qui contient des ingrédients ayant des effets bénéfiques pour la santé ou capables d'améliorer les fonctions physiologiques.

Par complément alimentaire, on entend une denrée alimentaire ayant pour but de compléter le régime alimentaire normal. Un complément alimentaire constitue une source concentrée de nutriments ou d'autres substances ayant un effet nutritionnel ou physiologique, lorsqu'ils sont pris seuls ou en combinaison, en de faibles quantités.

Par dentées alimentaires destinées à une alimentation particulière (DDAP), on entend un aliment ayant un objectif nutritionnel particulier, destiné à un groupe de population bien défini, tel que les nourrissons, les enfants en bas âge, les sportifs.

La composition alimentaire telle que mentionnée dans l'invention peut être un complément alimentaire ou une DDAP.

Les souches de l'invention ont été identifiées par la Demanderesse pour leurs nombreux avantages et notamment leur capacité à induire des effets bénéfiques sur le tractus digestif humain, en particulier l'intestin grêle et le colon mais aussi sur l'organisme en général.

En effet, il a été observé que, de manière surprenante, la levure ScPro1 et/ou SCB1 et/ou dérivé est capable d'induire une action anti-inflammatoire, contrairement à bon nombre de souches de levure, et ce, sans effet pro-inflammatoire

En effet, la levure ScPro1 et/ou SCB1 et/ou dérivé provoque l'augmentation de la sécrétion de l'interleukine IL-10 impliquée dans les signaux anti-inflammatoires. En outre, à la différence des actions des bactéries probiotiques de type lactobacilles, la levure ScPro1 et/ou SCB1 et/ou dérivé n'induit pas la synthèse de cytokine pro-inflammatoire IL-12. De même, la production des cytokines pro-inflammatoires $TNF\alpha$ et d'$IFN\gamma$ est nettement plus faible par rapport aux probiotiques bactériens. Des tests ont par ailleurs permis de démontrer l'effet anti-inflammatoire *in vivo* de la levure ScPro1, notamment une diminution de moitié de l'inflammation du gros intestin et une réduction d'un tiers de la nécrose intestinale.

En outre, la levure ScPro1 et/ou SCB1 et/ou dérivé, dans ses formes sèches, est capable de franchir la barrière gastrique sans aucun impact négatif sur sa survie ou son intégrité et cette levure ne s'implante pas dans un environnement colique.

La Demanderesse a démontré, pour la première fois et de manière particulièrement surprenante, que la levure ScPro1 et/ou SCB1 et/ou dérivé est capable d'augmenter la résistance à la douleur, notamment sur un modèle *in vivo* de rat.

En plus de ces effets bénéfiques, cette levure ScPro1 et/ou SCB1 et/ou dérivé est capable d'inhiber la colonisation et/ou l'invasion au niveau de l'intestin de microorganismes pathogènes et/ou à caractère invasif. L'administration de cette levure entraîne une diminution des entérobactéries au niveau du côlon et de la flore intestinale résistante aux antibiotiques.

En particulier, elle a montré une capacité prophylactique et thérapeutique à l'encontre de la colonisation intestinale par *Candida albicans* et des inflammations provoquées et entretenues par ce pathogène. Par ailleurs, cette levure présente un effet inhibiteur sur le pouvoir d'adhésion et d'invasion de souches pathogènes et/ou à caractère invasif de pathotypes *d'Escherichia coli* isolés de biopsies iléales de patients atteints par la maladie de Crohn.

Selon la présente invention, cette levure ScPro1 et/ou SCB1, en présence ou non d'un dérivé, peut être administrée sous une forme vivante ou revivifiable, de préférence par voie orale.

Par « forme vivante » ou « vivante », on entend, selon l'invention, une levure dont le métabolisme est actif ou réactivable ou capable de se multiplier. Il s'agit notamment de la levure sous forme sèche ou sous forme fraîche.

Typiquement, la levure sous forme fraîche se présente sous forme de levure pressée ou émiettée. Elle peut également se présenter sous forme de levure en suspension en phase aqueuse et on parle alors de levure liquide. Dans ce cas, la levure sera de préférence encapsulée. Les procédés d'encapsulation et les divers types de capsules sont bien connus de l'homme du métier.

Parmi les formes de levure sèche, on peut citer la levure pouvant se présenter sous la forme sèche instantanée ou sèche active. On entend par levure sèche, toute levure ayant un taux de matière sèche supérieur à 90%, de préférence allant d'environ 92% à 96%.

Parmi les levures sèches, on peut encore citer les levures à humidité intermédiaire, surgelée ou non.

La levure sèche instantanée est principalement destinée aux industriels et artisans de la boulangerie. D'autres applications et débouchés sont possibles sur la base des référentiels alimentaires (pharmacie, fermentation alcoolique). La particularité de cette levure sèche est de ne pas nécessiter de réhydratation avant d'être incorporée à la farine.

Elle provient de la déshydratation de la levure par l'action d'un gradient d'air chaud qui permet de transformer un produit pâteux (levure pressée ou liquide) en de fins vermicelles secs tout en restant active. Le produit doit ensuite, pour rester stable, être conditionné en absence d'oxygène.

La levure sèche active est une levure vivante, séchée à basse température, afin de lui conserver son pouvoir fermentatif et lui assurer une conservation très longue. Elle se présente sous forme de sphérules.

Cette levure provient de la déshydratation de la levure par l'action conjointe de la chaleur et d'une activité mécanique qui permettent de transformer la levure sous forme pâteuse en un produit sec en ménageant sa viabilité.

Les levures sèches actives sélectionnées sont obtenues par extrusion et séchage en lit fluidisé de la biomasse (cellules vivantes de levure). Cette levure sèche active, c'est-à-dire levure sèche ayant une teneur élevée en cellules vivantes de levure, se présente sous forme de granules avec généralement un diamètre de $0,1\mu m$ à 2,5 mm et une teneur en $H_2O$ de 4 à 8% en masse.

Ces formes sèches présentent l'avantage de procurer une meilleure gastro-résistance par rapport à la forme fraîche et optimisent les effets bénéfiques de la levure selon l'invention. La levure selon l'invention sera préférentiellement sous la forme d'une levure sèche active.

Il est généralement reconnu que les cytokines pro-inflammatoires stimulent les mécanismes inflammatoires qui peuvent alors être responsables de bon nombre de problèmes cliniques, en particulier dans les cas de maladies auto-immunes ou de déficiences immunitaires.

Ainsi, la levure selon la présente invention peut servir à prévenir et/ou traiter les maladies ou les troubles inflammatoires de l'intestin, qu'ils soient chroniques ou aiguës, éventuellement associés, ou non, à des diarrhées ou constipations.

Dans un premier mode de réalisation, les troubles et maladies sont associés à des diarrhées.

Dans un second mode de réalisation, les troubles et maladies ne sont pas associés à des diarrhées. Notamment, la levure ScPro1 et/ou SCB1 et/ou dérivé peut être utile pour la prévention ou le traitement des colites qui se caractérisent essentiellement par une inflammation du colon.

En particulier, cette levure est bien adaptée à la prévention et/ou au traitement des Maladies Inflammatoires Chroniques de l'Intestin (MICI), notamment la colite ulcéreuse, la rectocolite hémorragique, la maladie céliaque ou la maladie de Crohn.

Ces maladies se caractérisent notamment par une réponse immunitaire exacerbée dans laquelle sont impliquées de multiples cascades inflammatoires. Ainsi, dans le cadre de la prévention ou du traitement de ces maladies par un probiotique et/ou un alicament et/ou un aliment fonctionnel et/ou un nutraceutique et/ou un cosméceutique, il est important que les effets pro-inflammatoires soient les plus faibles possibles.

La levure ScPro1 et/ou SCB1 et/ou dérivé selon l'invention est donc tout particulièrement adaptée à ces utilisations. Cette levure possède plusieurs avantages supplémentaires.

Le premier est qu'elle possède la capacité d'augmenter la résistance à la douleur. Le deuxième, notamment pour la maladie de Crohn, est que cette levure est notamment capable d'inhiber le pouvoir d'adhésion et d'invasion des souches pathogènes et/ou à caractère invasif de *E. coli* provenant de patients souffrant de cette maladie.

La réponse inflammatoire peut être notamment due à l'invasion de tous microorganismes pathogènes.

Ainsi, la levure ScPro1 et/ou SCB1 et/ou dérivé selon l'invention montre une bonne efficacité pour prévenir ou traiter les désordres ou maladies gastro-intestinales dûs à la colonisation des intestins par des microorganismes pathogènes et/ou à caractère invasif procaryotes, tels que des bactéries, ou eucaryotes, tels que des champignons.

Les désordres ou maladies gasto-intestinales peuvent être des maladies inflammatoires chroniques intestinales, telles que la colite ulcéreuse, la maladie céliaque, la maladie de Crohn, la rectocolite hémorragique.

Par ailleurs, la levure ScPro1 et/ou SCB1 et/ou dérivé permettant d'augmenter la résistance à la douleur, elle présente également un intérêt dans le traitement préventif ou curatif de pathologies ou de troubles des intestins caractérisés par un état d'hyperalgésie. Ces pathologies ou troubles peuvent être notamment des troubles fonctionnels intestinaux, des maladies inflammatoires chroniques de l'intestin (MICI), ou des intolérances alimentaires (allergies, conditionnements, etc.....) caractérisées par une douleur viscérale chronique.

Elle est particulièrement adaptée au traitement préventif ou curatif des hyperalgésies et en particulier du syndrome de l'intestin irritable (SII) quelque soit sa forme (constipation, diarrhée ou une combinaison des deux), mais également de douleurs viscérales chroniques n'entrant pas dans le cadre du SII, telles que les douleurs abdominales fonctionnelles sans trouble de l'élimination fécale (FAPS : Functional Abdominal Pain) et les douleurs liées aux intolérances alimentaires et à la maladie coeliaque.

La levure ScPro1 et/ou SCB1 et/ou dérivé ou toute composition la comprenant, peut donc être utilisée de manière préventive chez des sujets présentant des prédispositions ou une sensibilité à ce type de désordres ou de maladies, ou de manière curative, par exemple lors de crises ou sur des périodes plus longues. Les compositions et méthodes de l'invention peuvent réduire la souffrance des sujets, d'atténuer les symptômes ou la cause de ces désordres.

La conjonction des effets de cette levure et/ou dérivé selon l'invention sur la douleur, l'inflammation et les microorganismes pathogènes et/ou à caractère invasif provoque de manière certaine cette amélioration du bien-être, de la santé et/ou du confort du tractus gastro-intestinal humain ou animal.

La composition peut comprendre une levure ScPro1 et/ou une levure SCB1 et/ou au moins un dérivé de levure *Saccharomyces cerevisiae* choisi parmi les extraits de levure, les dérivés de parois, les glucanes pariétaux, les mannoprotéines pariétales, les fractions lipidiques de levure, les fractions d'acides nucléiques de levure (ARN, ADN) en une quantité allant d'environ entre $10^7$ et $6.10^{10}$UFC, et de préférence entre $10^8$ et $2.10^{10}$UFC, ou, entre 1 mg et 10 g, et de préférence entre 1 mg et 1g. Cette quantité peut être une quantité journalière prise en une ou plusieurs fois au cours de la journée.

De manière préférentielle, la levure ScPro1 et/ou SCB1 et/ou dérivé est utilisée dans les applications thérapeutiques ou non thérapeutiques en une dose journalière comprise entre $10^7$ et $6.10^{10}$ UFC (Unités Formant Colonie), et de préférence entre $10^8$ et $2.10^{10}$ UFC.

Dans le cas où la levure est sous une forme vivante mais non revivifiable, la dose journalière utile dans les applications thérapeutiques ou non thérapeutiques sera de préférence comprise entre 1 mg et 10 g, de préférence entre 1 mg et 1 g. La dose efficace journalière peut être administrée en une, deux, trois ou quatre fois.

La levure et /ou dérivé selon l'invention ou les compositions la comprenant sont de préférence administrées par voie orale. Elle peut l'être par quantité thérapeutiquement efficace, ce qui signifie qu'au moins un des symptômes est diminué ou supprimé.

La levure ScPro1 et/ou SCB1 et/ou dérivé peut être incluse dans une composition alimentaire humaine ou animale et/ou administrée avec des excipients ou véhicules appropriés à l'administration orale.

La composition destinée à l'alimentation humaine peut être un liquide, une pâte ou un solide. Notamment, la composition peut être un produit laitier tel que du fromage, du beurre, un yaourt ou une crème, un produit à base de fruit comme un jus de fruit, une compote ou une pâte de fruit, une boisson, ou un aliment solide, par exemple un snack, un biscuit, ou autre. Ainsi, la composition comprend la levure ScPro1 et/ou SCB1 et/ou dérivé et les composants de l'aliment ou de la boisson.

La levure ScPro1 et/ou SCB1 et/ou dérivé peut aussi être incluse dans une composition pharmaceutique. La composition pharmaceutique est adaptée à une administration orale. Elle comprend donc la levure ScPro1 et/ou SCB1 et/ou dérivé ainsi qu'un support classique adéquat choisi parmi les excipients autorisés pour la fabrication de préparation pharmaceutique. Elle peut être formulée sous forme liquide, comme un sirop ou une ampoule, ou sous forme de comprimés, de gélules, de sachets, de capsules ou de poudre et autres formes galéniques appropriées.

La levure ScPro1 et/ou SCB1 et/ou dérivé peut en outre être administrée avec d'autres probiotiques et/ou d'autres ingrédients fonctionnels, en particulier bactéries probiotiques notamment pour une action préventive encore plus complète.

On pourra citer à titre d'exemple les bactéries lactiques des genres Lactobacillus, Bifidobacterium, Pediococcus, Propionibacterium, ou Leuconostoc.

La levure ScPro1 et/ou SCB1 et/ou dérivé peut également être administrée avec d'autres principes actifs tels que des antibiotiques, des analgésiques, des anti-diarrhéiques, des laxatifs, et leurs mélanges.

La présente invention va maintenant être illustrée à l'aide des exemples et des figures qui suivent, qui sont donnés à titre d'illustration, ne sont nullement limitatifs, et dans lesquelles figures :

- la figure 1 représente le suivi de la survie de la levure ScPro1 dans un système digestif artificiel simulant le côlon humain, selon l'exemple 2,
- la figure 2 représente les effets de la levure ScPro1 sur la microflore colique, selon l'exemple 2,
- les figures 3 et 4 représentent l'évolution du nombre de cellules de *Candida albicans* dans des selles de souris pour les expérimentations 1 et 2 de l'exemple 5 correspondant aux modèles prophylactique (figure 3) et curatif (figure 4),

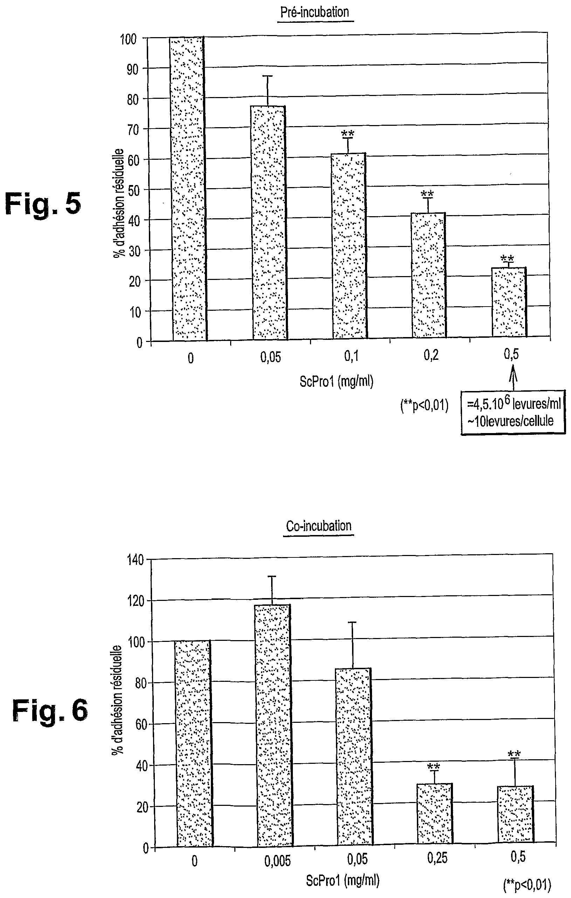

- la figure 5 représente le pourcentage d'adhésion résiduelle des cellules *d'Escherichia coli* AIEC LF82 aux cellules épithéliales intestinales humaines en fonction de la quantité de levure ScPro 1 avec pré-incubation ; les cellules de levure ont été incubées avec les cellules épithéliales intestinales pendant une heure. L'infection des cellules avec la souche AIEC LF82 a été effectuée en présence de la levure ScPro1, selon l'exemple 6,
- la figure 6 représente le pourcentage d'adhésion résiduelle des cellules *d'Escherichia coli* AIEC LF82 aux cellules épithéliales intestinales humaines en fonction de la quantité de levure ScPro1 avec co-incubation ; les cellules de levure et les cellules *d'Escherichia coli* ont été incubées simultanément avec les cellules épithéliales intestinales pendant une heure selon l'exemple 6,
- la figure 7 représente l'estimation de l'intensité de l'inflammation des intestins de souris selon le score macroscopique de Wallace après administration des levures ScPro1 et SCB1, selon l'exemple 4,
- la figure 8 représente l'estimation de l'intensité de l'inflammation de l'épithélium intestinal d'intestin de souris selon le score histologique d'Ameho après administration des levures ScPro1 et SCB1, selon l'exemple 4,
- la figure 9 représente l'estimation de l'intensité de l'inflammation des intestins de souris selon le score macroscopique de Wallace après administration des levures ScPro1 et SCB1 prises seule ou en combinaison, selon l'exemple 4,
- la figure 10 représente l'estimation de l'intensité de l'inflammation de l'épithélium intestinal de souris selon le score histologique d'Ameho après administration des levures ScPro1 et SCB1, prises seules ou en combinaison, selon l'exemple 4,
- la figure 11 représente le niveau d'expression d'ARNm du gène codant pour la protéine IL-10 une heure, et trois heures après de la mise en contact des levures ou dérivés selon l'invention avec les cellules épithéliales intestinales humaines selon l'exemple 7,
- la figure 12 représente le niveau d'expression d'ARNm du gène codant pour le récepteur nucléaire PPAR$\alpha$ une heure après, et trois heures après la mise en contact des levures ou dérivés selon l'invention avec les cellules épithéliales intestinales humaines selon l'exemple 7,
- la figure 13 représente la modulation de l'expression d'ARNm du gène codant pour la protéine IL-10 après une heure, et trois heures après la mise en contact des dérivés de levure selon l'invention avec les cellules épithéliales intestinales humaines selon l'exemple 7,
- la figure 14 représente l'expression du gène codant pour la protéine IL-10 dans des cellules épithéliales intestinales de souris après administration de la levure et/ou dérivé selon l'invention (exemple 4),
- la figure 15 représente l'expression du gène codant pour le récepteur nucléaire PPAR$\alpha$ dans des cellules épithéliales intestinales de souris après administration de levure et/ou dérivé selon l'invention (exemple 4),
- la figure 16 montre les quantités de cytokine IL-10 sécrétées, mesurées en pg/ml, par les cellules intestinales de biopsies de patients atteints ou non de la maladie de Crohn après leur mise en contact avec des levures et/ou dérivés selon l'invention (exemple 8),
- la figure 17 montre les quantités de cytokine TNF-$\alpha$ sécrétées, mesurées en pg/ml, par les cellules intestinales de biopsies de patients atteints ou non de la maladie de Crohn après leur mise en contact avec des levures et/ou dérivés selon l'invention (exemple 8),
- la figure 18 montre le résultat de l'essai de détermination de la capacité de liaison de pili de type 1 sur des fractions mannoprotéiques (EL 05 et EL 06) de levure ScPro1,
- les figures 19A et 19B montrent, respectivement, les pourcentages d'adhésion et d'invasion résiduelle moyens de la souche AIEC LF82 par rapport aux cellules T84, lors d'une co-incubation avec des concentrations croissantes de levure (exemple 6) - * $p < 0,05$, **$p < 0,01$,
- les figures 20A et 20B montrent, respectivement, les pourcentages d'adhésion et d'invasion moyens de la souche AIEC LF82 par rapport aux cellules T84 lors d'une co-incubation avec des concentrations croissantes de mannoprotéines de levure EL05 -(exemple 6),
- les figures 21A et 21B montrent, respectivement, les pourcentages d'adhésion et d'invasion résiduelle moyens de la souche AIEC LF82 par rapport aux cellules T84 lors d'une pré-incubation avec des concentrations croissantes de levure (exemple 6) - *p<0,05 et **p<0,01,

- les figures 22A et 22B montrent, respectivement, les pourcentages d'adhésion et d'invasion moyens de la souche AIEC LF82 par rapport aux cellules T84 lors d'une pré-incubation avec des concentrations croissantes de mannoprotéines de levure EL05 (exemple 6) - *p<0,05 et **p<0,01,
- la figure 23 montre les pourcentages d'adhésion résiduelle de la souche AIEC LF82 par rapport aux cellules CHO-K1 et CHO-K1/CEACAM6, lors d'une pré-incubation avec des concentrations croissantes de levure ScPro1 sèche instantanée (exemple 6) - *p<0,05 et**p<0,01,
- la figure 24 montre l'adhésion de la souche AIEC LF82 ou du mutant non pilié LF82-δfimH à la bordure en brosse d'entérocytes de 3 prélèvements de cellules atteints de la maladie de Crohn (exemple 6),
- la figure 25 montre la mesure des seuils de perception de la douleur (mesurés en mm de mercure) par rapport à différentes levures sur des rats sains, (exemple 9), et
- la figure 26 montre la mesure des seuils de perception de la douleur (mesurés en mm de mercure) par rapport à différentes levures sur des rats ayant une hypersensibilité viscérale (exemple 9).

## EXEMPLES :

## EXEMPLE 1 : Survie de la levure ScPro1 et/ou SCB1 dans un environnement digestif artificiel simulant l'intestin humain

### Etude du devenir de la levure ScPro1 et /ou SCB1 au cours du transit gastro-intestinal

Les levures ScPro1 et SCB1 ont été testées et étudiées *in vivo* dans un environnement digestif artificiel simulant la digestion humaine et notamment par l'étude de la survie des levures testées viables au cours du transit gastro-intestinal.

Deux échantillons de la forme levure sèche active ScPro1 et deux échantillons de la forme levure sèche active SCB1 ont été testés.

Les deux échantillons se différencient par le temps de stockage à température ambiante sous vide: soit vieillissement inférieur à 6 mois, soit vieillissement de 2 ans.

• *Conditions expérimentales :*

Les digestions ont été réalisées dans le système dénommé TIM1 (estomac + intestin grêle), suivant des conditions expérimentales établies à partir de données issues de la littérature et reproduisant la digestion d'un aliment liquide (eau) chez l'homme adulte sain à jeun, avec élimination des produits de digestion par dialyse et absorption. Chaque digestion a été conduite sur 5 heures. Toutes les digestions ont été réalisées dans les mêmes conditions opératoires générales à savoir :

*Température :* La température a été de 37°C.
*Paramètres de vidange gastrique :* La vidange gastrique suit la loi définie par Elashoff *et al.* (1982) énoncée comme suit :

$$F = 1 - 2^{-(t/T)^b}$$

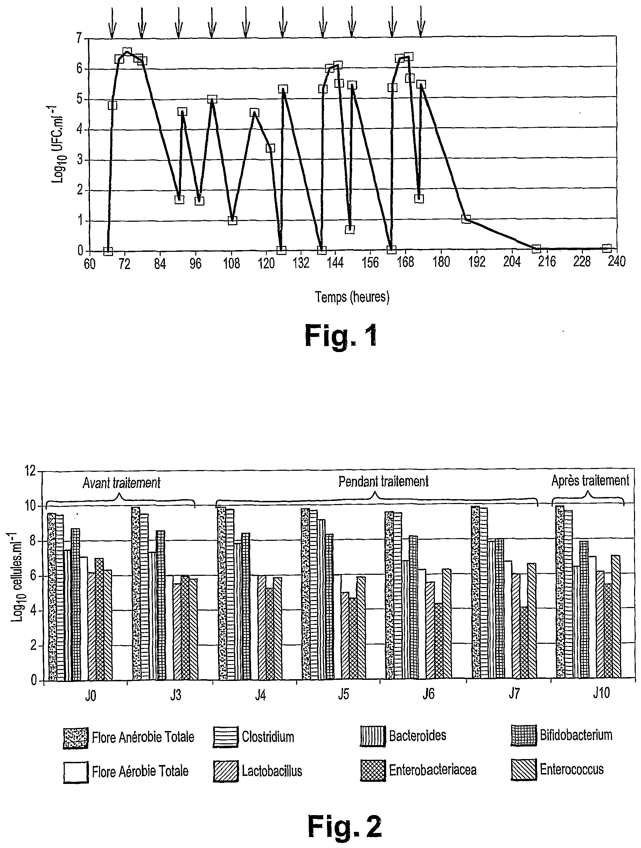

où F représente la fraction de repas délivrée, t le temps, T le temps de demi-vidange de l'aliment et b un paramètre décrivant l'allure de la courbe. Les paramètres sont T = 15 min ; b = 1.
*Paramètres de vidange iléale :* La vidange iléale suit la loi d'Elashoff modifiée (introduction d'un paramètre d permettant le ralentissement de la vidange en fin de digestion, $F_m = F + d * t^3$). Les paramètres sont : T = 150 min ; b = 2,4 ; d = $-10^{-7}$ (*cf.* Figure 2).
*Consignes de pH :*

Estomac (min/pH) : 0/6,0 ; 10/3,2 ; 20/2,4 ; 40/1,8 ; 60/1,6 ; 90/1,5 ; 300/1,5
Duodénum : 6,4
Jéjunum : 6,9
Iléum : 7,2

*Sécrétions gastriques :*

HCl
Pepsine
Lipase

*Sécrétions intestinales :*

NaHCO$_3$ dans les trois parties de l'intestin grêle
Extrait de bile dans le duodénum
Extrait pancréatique dans le duodénum

*Dialyse/Absorption :*

L'élimination des "petites" molécules du chyme intestinal a été réalisée à deux niveaux du TIM1 (le jéjunum et l'iléon) à l'aide d'hémodialyseurs. La dialyse du chyme intestinal a été réalisée en continue contre une solution saline dont la composition était proche de celle du plasma sanguin. Les dialysats ont été collectés dans des sacs de dialyse.

Des prélèvements ont été réalisés au cours de la digestion à différents niveaux du tractus afin de suivre la survie des levures testées.

Les dénombrements de levures ont été réalisés conformément aux méthodologies microbiologiques classiques et ont été effectués sur les prélèvements pratiqués, dans l'estomac à 10, 20, 30 et 45 min, dans les sorties iléales cumulées sur des périodes d'une heure, et dans le résidu final.

La méthode de dénombrement était la suivante :

Chaque prélèvement a subi rapidement une dilution sériée au dixième dans de l'eau physiologique (NaCl 8,5 g/l) stérile. Puis 0,1 ml de chaque dilution a été déposé puis étalé en surface d'un milieu gélosé réparti en boîte de Pétri (deux boîtes par dilution). Les boîtes ont été incubées 48 h à 35°C avant de procéder à la numération des "Unités Formant Colonies" (UFC).

Le résultat des numérations a été exprimé en UFC/ml (données brutes) et en pourcentage de cellules de levures vivantes par rapport au nombre de levures initialement introduites, afin de déterminer les taux de survie des levures dans l'estomac et en sortie de l'intestin grêle.

Le tableau suivant résume les taux de survie théoriques (si viabilité de 100%) et réels obtenus pour chaque souche aux niveaux de l'estomac, de l'ensemble des sorties iléales après 5 h de digestion, et de l'ensemble du système après 5 h de digestion.

• **Résultats :**

| Produits digérés | Levures introduites en UFC | Sortie estomac à T=45min | Sortie iléale à T=5h | Survie globale à T=5h |
|---|---|---|---|---|
| Scpro1 lot1 | $3.5\ 10^{10}$ | 89% | 100% | 106% |
| Scpro1 lot2 | $2.0\ 10^{10}$ | 88% | 95% | 106% |
| SCB1 1 | $1.5\ 10^{10}$ | 83% | 76% | 81% |
| SCB1 2 | $1.5\ 10^{10}$ | 85% | 69% | 76% |

• **Conclusion**

Ces résultats démontrent bien une excellente survie gastro-intestinale pour les levures ScPro1 et SCB1.

**EXEMPLE 2 : Survie de la levure ScPro1 dans un environnement digestif artificiel simulant l'intestin humain**

**Etude de la survie de la levure ScPro1 pendant la fermentation colique et de leur influence sur la microflore intestinale**

La levure ScPro1 sous forme sèche active a été testée et étudiée in vitro dans un environnement digestif artificiel simulant la digestion humaine et notamment par l'étude du devenir et de l'impact environnemental de levures testées viables durant la fermentation colique.

La fermentation colique s'apparente à une fermentation continue avec des apports séquencés de milieu pour le maintien de la flore. Ce milieu renferme principalement des composés glucidiques complexes et non digérés dans la partie haute du tractus digestif (amidon, pectine, cellulose...), des composés protidiques plus ou moins hydrolysés et de la mucine.

Du milieu colique est retiré du fermenteur également de façon séquencée. Le milieu est parcouru par un système de dialyse qui permet l'élimination continue des produits solubles de fermentation.

Le dialysat est collecté pour analyse des acides gras à chaînes courtes (AGCC). Le milieu est maintenu en anaérobiose créée par les propres gaz de fermentation et il présente un potentiel d'oxydo-réduction inférieur à -300 mV. Enfin le pH est régulé avec un point de consigne de 6.

Chaque digestion a comporté : une période de stabilisation de la flore de 2-3 jours après ensemencement du côlon, une période de traitement (au moins 3 jours) avec au moins un ajout quotidien de produit, et une période d'arrêt du traitement de 3 jours. A chaque expérience, les paramètres suivant ont été suivis et/ou enregistrés :

- la viabilité des levures,
- l'évolution de différentes populations bactériennes aérobie et anaérobie,
- l'évolution des principaux produits de fermentation (AGCC et gaz),
- la détection d'activités enzymatiques standards, et
- la température, le pH et le potentiel d'oxydo-réduction.

La fermentation a été réalisée dans un flacon à pénicilline de 60 ml, fermé par un septum serti, sur 30 ml de milieu colique (milieu de culture plus flore fécale fraîche). L'échantillon de levure a été ajouté aux 30 ml de milieu.

Le milieu colique était constitué d'une part d'une suspension microbienne issue de selles fraîches dans un tampon phosphate et d'autre part d'un aliment type, également utilisé pour la culture de la flore colique dans le côlon artificiel.

Après mélange du milieu colique avec le produit à tester, le flacon a été bouché et serti.

Toutes ces manipulations ont été réalisées dans une hotte anaérobie (mélange de gaz, sans oxygène). Les flacons ont été placés en incubateur rotatif (37°C - 200 rpm) pendant 24 h.

Pour chaque produit, le test a été dupliqué. Par ailleurs, 4 flacons témoins (sans produit) ont été préparés dans les mêmes conditions. Deux flacons ont été traités immédiatement (temps initial) et deux flacons ont été incubés comme les flacons tests.

Les fermentations ont été arrêtées à 24 h et les flacons ont alors été traités.

**Production de gaz fermentaires :** Le volume de gaz produit par fermentation a été déterminé à l'aide d'un système de Mariotte (principe de la mesure du déplacement d'eau chassée par le gaz sous pression contenu dans le flacon à pénicilline). Une analyse des gaz présents dans le flacon a alors été réalisée par CPG ($H_2$, $CO_2$, $CH_4$, $O_2$).

**Production d'acides gras à chaînes courtes :** Un premier prélèvement du contenu colique a été effectué. Il a été soit congelé, soit directement traité afin de déterminer les concentrations en AGCC (acides gras volatiles à courtes chaînes) du surnageant de culture. Cette analyse a été réalisée par CPG. Les métabolites recherchés ont été les acides : acétique, propionique, butyrique, isobutyrique, valérique, isovalérique, caproïque, isocaproïque et heptanoïque.

**Analyse microbiologique :** Un deuxième prélèvement du contenu colique a été effectué et traité immédiatement (dilution sériée au dixième dans un milieu de dilution réduit) afin de dénombrer : la flore anaérobie totale, la flore aéro-anaérobie facultative et la flore fongique.

Les résultats concernant la survie de la levure ScPro1 sont représentés sur la figure 1. Sur cette figure, chaque flèche verticale indique l'administration de levure ScPro1.

Il a été constaté que la levure ScPro1 montre une bonne survie au 3ème jour après l'administration et une forte mortalité entre le 4ème et le 7ème jour lors période d'administration. Ceci montre que cette levure ne s'implante pas dans un environnement colique.

Les résultats de l'analyse microbiologique sont représentés sur la figure 2. Ils montrent la diminution des entérobactéries en présence de la levure ScPro1 avec une remontée après l'arrêt de l'administration de la levure. Lors de l'administration de la levure ScPro1, il a également été constaté que la flore résistante aux antibiotiques (chloramphénicol, gentamycin) diminue significativement.

Les résultats concernant l'effet de la levure ScPro1 sur la production d'acides gras volatils à courtes chaînes (AGCC) sont résumés dans le tableau ci-dessous (exprimés en mM dans le milieu colique).

|  | Avant traitement | Pendant traitement | Après traitement |
|---|---|---|---|
| Acétate | $71,4 \pm 2,3$ | $57,6 \pm 4,2$ | $60,6 \pm 0,7$ |
| Propionate | $22,8 \pm 0,6$ | $26,5 \pm 4,2$ | $35,7 \pm 1,1$ |
| Butyrate | $35,0 \pm 1,6$ | $36,5 \pm 2,2$ | $26,6 \pm 4,2$ |
| Iso-Butyrate | $3,2 \pm 0,3$ | $3,3 \pm 0,2$ | $3,4 \pm 0,1$ |
| Iso-Valérate | $5,6 \pm 0,5$ | $5,2 \pm 0,2$ | $5,3 \pm 0,0$ |
| Valérate | $8,0 \pm 0,6$ | $7,8 \pm 1,4$ | $9,1 \pm 0,9$ |
| Iso-Caproate | $0,1 \pm 0,1$ | $0,0 \pm 0,0$ | $0,0 \pm 0,0$ |
| Caproate | $9,0 \pm 1,1$ | $7,3 \pm 0,3$ | $5,7 \pm 0,7$ |
| Heptanoate | $0,2 \pm 0,2$ | $0,0 \pm 0,1$ | $0,0 \pm 0,0$ |
| Total | $155,3 \pm 2,7$ | $144,1 \pm 6,8$ | $146,4 \pm 3,4$ |

Durant le traitement, il a été constaté une diminution de l'acétate, en partie au profit du propionate, ce qui suggère une diminution de l'activité de la microflore acétogène.

Parmi les autres paramètres suivis, il n'a pas été observé d'effet notoire du traitement sur

✔ La production de gaz (en quantité et en proportion);
✔ Les concentrations en sucres totaux et simples (stables dans le temps); et
✔ Les activités enzymatiques.

**EXEMPLE 3: Etude de l'influence de la levure ScPro1 ou SCB1 sur l'induction de la production de cytokines**

On a étudié l'influence des levures vivantes ScPro1 et SCB1 sur l'induction de la production de cytokines sur des cellules périphériques mononucléaires sanguines humaines (PBMC).

Les levures ScPro1 et SCB1 ont été testées dans leur forme sèche instantanée et sèche active sur le plan de leur capacité à induire la production des cytokines IL-10, IL-12, TNF$\alpha$, TNF$\gamma$ chez les PBMC humaines.

Préparation des cellules périphériques mononucléaires sanguines humaines

Du sang humain frais, obtenu à partir de sujets en bonne santé au Centre de Transfusion, a été dilué 2 fois avec du PBS-Ca (GIBCO) et purifié sur un gradient Ficoll (GIBCO). Après centrifugation à 400 x g pendant 30 minutes à 20°C, les cellules mononucléaires sanguines périphériques (PBMC) formaient une couche circulaire dans le sérum. Les PBMC ont été soigneusement aspirées, mises en suspension dans un volume final de 50 ml utilisant du PBS-Ca et lavées 3 fois dans la même solution tampon avec des étapes de centrifugation de 10 minutes à 20°C à 350 x g. Les PBMC ont ensuite été remises en suspension en utilisant un milieu RPMI complet (GIBCO), enrichi avec 10 % w/v de sérum de veau foetal (inactivé à 56°C pendant 30 minutes), 1 % w/v de L-glutamine (GIBCO) et de la gentamycine (150 $\mu$g/ml) (GIBCO). Les PBMC ont été dénombrées au microscope, ajustées à une concentration de $2 \times 10^6$ cellules / ml et réparties (dans 1 ml de la solution aliquote) sur des boites de culture cellulaires de 24 puits (Corning, Inc.).

Préparations microbiologiques

Des cultures de Lactobacillus, Lactococcus et de Escherichia coli (souches de contrôles), réalisées pendant la nuit, ont été lavées 2 fois avec un tampon PBS à pH 7.2, avant d'être remises en suspension dans du PBS à une concentration de $2.10^9$ UFC/ml.

La concentration de levure utilisée dans les premières expérimentations était de $2.10^8$ ufc/ml. Pour une étude de comparaison de dose initiale, des dilutions sérielles de 10 en 10 pouvaient être faites pour comparer les effets de $2.10^7$ UFC/ml, $2 \times 10^8$ UFC/ml et $2.10^9$ UFC/ml.

Incubation des cellules périphériques mononucléaires sanguines humaines

10 µl de ces suspensions de travail ont été transférées dans les puits des boites contenant les PBMC, qui ont été mises à incuber à 37°C dans un mélange gazeux composé de 5 % de $CO_2$ et 95 % d'air atmosphérique. Après 24 heures d'incubation, le surnageant a été aspiré, centrifugé à 2 000 t/min (modèle Eppendorf), retiré et conservé à -20°C.

Le contrôle se compose de bactéries à Gram positif (Lactobacillus et Lactococcus), une bactérie à Gram négatif (*Escherichia coli*) et tampon sans levure.

Quantification des cytokines

Les niveaux d'expression des cytokines ont été déterminés par ELISA. Les plaques ELISA ont été tapissées d'un anticorps (pendant une nuit) et l'anticorps a été saturé avec du PBS / BSA 1% (serum albumine bovine). Un étalonnage a été préparé avec des concentrations connues de cytokines, avec un seuil de détection de 15,62 à 2000 pg/ml (incubation sur la nuit). La recherche et la quantification d'anti-cytokine ont été réalisées par mesure de l'activité streptavidine avec le substrat TMB (tétraméthylbenzidine, Pharmingen2). Les kits Pharmingen du commerce ont été utilisés en conformité avec la description du fabriquant. Quatre cytokines ont été choisies : 3 pro-inflammatoires (TNF$\alpha$, IFN$\gamma$, IL-12) et une anti-inflammatoire (IL-10).

Résultats

Les réponses des 4 cytokines sur 5 donneurs distincts ont été évaluées au ratio 1/1, levures/ PBMC.

Les résultats des dosage des 4 cytokines sécrétées dans le surnageant de culture sont résumés dans le tableau A ci-dessous. Les données sont exprimées comme la valeur moyenne (Moy) issues des dosages des 5 donneurs. Le tableau donne également la valeur (Sem) de l'erreur standard sur la moyenne.

Tableau A

|  | IL-10 (pg/ml) | | IFN$\gamma$ (pg/ml) | | TNF$\alpha$ (pg/ml) | | IL-12 (pg/ml) | |
|---|---|---|---|---|---|---|---|---|
|  | Moy | Sem | Moy | Sem | Moy | Sem | Moy | Sem |
| **Témoin négatif** | 0 | 0 | 50 | 0 | 50 | 0 | 0 | 0 |
| **E. coli** | 2474 | 839 | 57376 | 29591 | 11185 | 3875 | 15 | 15 |
| **Lactococcus lactis** | 111 | 43 | 136103 | 62706 | 25362 | 9818 | 1101 | 543 |
| **Bifidobacterium longum** | 1072 | 355 | 33780 | 27164 | 14517 | 5601 | 22 | 20 |
| **Lactobacilus acidophilus** | 435 | 259 | 85543 | 46838 | 18369 | 6857 | 529 | 343 |
| **SCB1** | 569 | 291 | 27807 | 19231 | 6492 | 2698 | 14 | 10 |
| **ScPro1** | 442 | 292 | 15218 | 9304 | 3643 | 1847 | 8 | 5 |

Il a été observé :

1) Pour les levures ScPro1 et SCB1, la production de quantités très faibles, voire indétectables, d'IL-12 induite par les PBMCs, contrairement aux bactéries de référence.

2) Des niveaux substantiels d'IL-10 à la fois pour les levures vivantes laissant apparaître que SCB1 présente un meilleur résultat que ScPro1.

3) En ce qui concerne l'IFN$\gamma$ et le TNF$\alpha$, les quantités sécrétées sous l'action des levures ScPro1 et SCB1 sont nettement plus faibles comparativement avec les différentes bactéries probiotiques testées.

Conclusions :

- Il apparaît clairement que Les levures ScPro1 et SCB1 en présence de PBMC n'induisent pas la cytokine pro-inflammatoire IL-12, contrairement à ce qui est classiquement observé avec les lactobacilles probiotiques.
- Les levures ScPro1 et SCB1 en présence de PBMC induisent des niveaux substantiels d'IL-10 (anti-inflammatoire).
- Les quantités sécrétées d'IFN-$\gamma$ et de TNF-$\alpha$ par les PBMC en présence des levures ScPro1 ou SCB1 sont nettement plus faibles qu'avec les bactéries probiotiques.

### Exemple 4 : Evaluation de l'effet protecteur des levures ScPro1 et SCB1 vis-à-vis de la colite sur un modèle chimio-induit (TNBS) de souris

Le modèle animal proposé est couramment utilisé et a été adapté pour mesurer les effets anti-inflammatoires des levures.

Des souris Balb/c âgées de 6 semaines ont été utilisées au cours de cet essai. Les souris ont été acclimatées aux conditions du laboratoire une semaine avant l'expérimentation, avec eau et nourriture fournies ad libitum. Chaque échantillon a été testé sur un groupe de 10 souris. Les colites ont été induites par un cycle de distribution d'eau de boisson *ad libitum* contenant 5% (w/v-1) de TNBS pendant 7 jours. Les levures ont été administrées oralement par gavage une fois par jour, 3 jours avant le début de l'induction de la colite par le TNBS et pendant la durée du traitement au TNBS (7 jours).

En plus des deux groupes testés, on a eu recours à un groupe témoin (contrôle négatif) pour lequel on n'a utilisé qu'une solution physiologique.

Les paramètres testés sont les suivants à l'issue des traitements :

- **L'évaluation macroscopique de l'inflammation intestinale (Score de Wallace).** Le colon de chaque souris a été examiné sous un microscope à dissection (agrandissement, x5) pour évaluer les lésions macroscopiques selon le système de score de Wallace qui va de 0 à 10 en fonction de critères d'évaluation révélateurs de la sévérité de l'inflammation tels que l'hyperémie, l'épaisseur des parois du colon et l'étendue des ulcérations.
- **L'évaluation histologique de l'inflammation (Score d'Ameho).** Une section du colon prélevée exactement à 2 cm du canal anal a été utilisée pour réaliser l'évaluation histologique selon le score d'Ameho qui va de 0 à 6 selon le degré d'infiltration de l'inflammation, la présence d'érosion, d'ulcérations ou de nécroses et la profondeur ainsi que l'extension en surface des lésions. La quantification des dégradations et des lésions intestinales a été effectuée par 2 opérateurs indépendants.
- **La quantification des l'expression des gènes codant pour IL-10 et PPAR$\alpha$** Pour ce faire, l'ARN total a été isolé des tissus coloniques au moyen du kit RNeasy (Macherey Nagel, Hoerdt, France) selon les instructions du fabricant. La quantification de l'ARN messager a été effectuée en utilisant un spectrophotomètre. Après un traitement à 37°C pendant 30 minutes avec 20-50 unités de DNase 1 RNase-free (Roche Diagnostics Corporation, Indianapolis, IN, USA), des primers oligo-DT (Roche Diagnostics Corporation, Indianapolis, IN, USA) ont été employés pour synthétiser les ADN circulaires simple brin. Les ARN messagers ont été quantifiés avec le SYBR green Master Mix (Applera, Courtaboeuf, France) et avec des oligonucléotides humains spécifiques pour études in vitro (voir tableau B ci-dessous), au moyen de l'appareil GeneAmp Abiprism 700 (Applera, Courtaboeuf, France). Des contrôles calibrés et non calibrés ont été inclus dans chaque essai. Chaque échantillon a été mesuré trois fois. L'intensité de la couleur du SYBR vert a été analysée avec le logiciel Abiprism 7000 SDS (Applera, Courtaboeuf, France). Tous les résultats seront normalisés par rapport au gène codant pour la $\beta$-actine.

Tableau B

| Gènes | Séquences d'amorces nucléotidiques |
|---|---|
| $\beta$-actin | F: 5'- AAgTCCCTCACCCTCCCAAAAg -3'<br>R : 5'- AAgCAATgCTgTCACCTTCCC-3' |
| PPAR$\alpha$ | F: 5'- ACgATgCTgTCCTCCTTgATg -3'<br>R: 5'- gTgTgATAAAgCCATTGCCgT -3' |
| IL-10 | F: 5'-CAgTCAgCCAgACCCACAT-3'<br>R: 5'-gCTCCACTgCCTTgCTTT-3' |

Les levures ScPro1 et SCB1 ont été testées dans le modèle préventif standard décrit ci-dessus. Un suivi pondéral des animaux avant l'induction de la colite a montré que les préparations de levure administrées aux souris ont été très bien tolérées.

L'inflammation intestinale, estimée par le score de Wallace, a été réduite de 60% avec les levures ScPro1 (sèche active, 1 mg/jour) et SCB1 comparé au contrôle positif. La levure SCB1 a également induit une réduction de l'inflammation. De même, la nécrose intestinale, estimée par le score Ameho a été réduite d'un tiers avec la levure ScPro1 (sèche instantanée, 1 mg ou 100 $\mu$g/jour) comparé au contrôle positif.

Les levures ScPro1 et SCB1, administrées seules ou ensemble, ont augmenté le niveau d'expression des gènes codant pour l'interleukine anti-inflammatoire IL-10 et le récepteur nucléaire PPARα

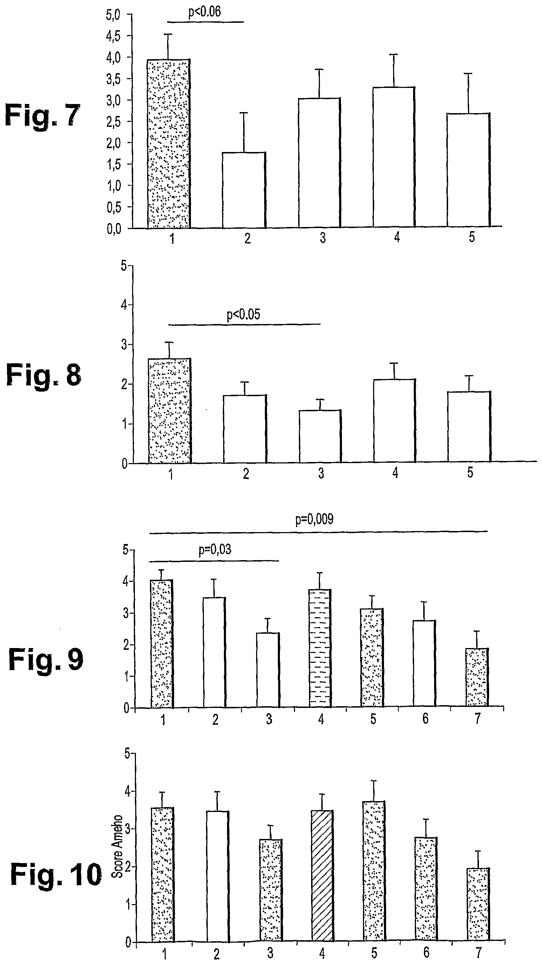

Les figures 7 à 10 illustrent bien les excellentes valeurs de scores macroscopiques de Wallace et Ameho des levures ScPro1 et SCB1 à des dosages journaliers différents.

Sur les figures 7 et 8, on a représenté, respectivement, le score macroscopique de Wallace et le score histologique d'Amého des levures ScPro1 et SCB1 sous forme sèche instantanée, dosée quotidiennement à 10 μg et 1 mg.

Les chiffres de chaque colonne du graphe des figures 7 et 8 représentent les éléments suivants:

1 représente le TNBS seul,
2 représente TNBS + ScPro 1 (1 mg),
3 représente TNBS + ScPro1 (100 μg),
4 représente TNBS + SCB1 (1 mg),
5 représente TNBS +SCB1 (100 μg).

On peut noter que la levure ScPro1 sèche instantanée, dosée à 100 μg/jour, réduit de manière significative les lésions aux niveaux macroscopique et histologique.

Sur les figures 9 et 10, on a représenté, respectivement, le score macroscopique de Wallace et le score histologique d'Amého des levures ScPro1 et SCB1, prises seules et en combinaison, sous forme sèche instantanée ou sèche active, dosées quotidiennement à 100 μg et 1 mg.

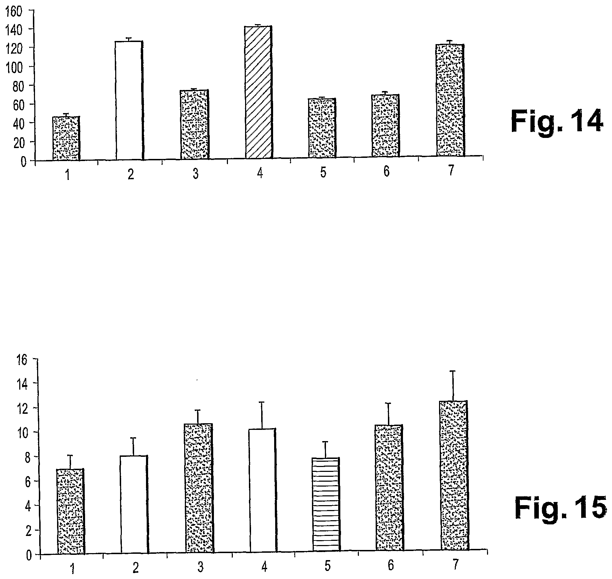

Les figures 14 et 15 montrent, respectivement, le niveau d'expression des gènes codant pour l'interleukine anti-inflammatoire, et pour le récepteur nucléaire PPARα au niveau des cellules intestinales.

Les chiffres de chaque colonne du graphe des figures 9, 10, 14 et 15 représentent les éléments suivants:

1 représente le TNBS seul,
2 représente TNBS + ScPro 1 sèche instantanée (100 μg),
3 représente TNBS + ScPro1 sèche active (100 μg),
4 représente TNBS + SCB1 sèche active (100 μg),
5 représente TNBS +ScPro1 sèche active (1 mg),
6 représente TNBS + ScPro1 sèche active (100 μg),
7 représente TNBS + ScPro1 sèche active (100 μg) + SCB1 (100 μg).

Conclusions :

On peut noter que ScPro1 sous forme sèche active induit de manière significatives les lésions au niveau macroscopique, et qu'un effet anti-inflammatoire synergique existe pour la combinaison de ScPro1 et SCB1, tant au niveau macroscopique qu'au niveau histologique.

ScPro1 et SCB1 ont augmenté respectivement par 2,9 et 3,1 le niveau d'expression du gène codant pour l'interleukine anti-inflammatoire IL-10 à des doses de 100 μg. La combinaison ScPro1 + SCB1 (histogramme n°7) multiplie par 2,7 ce niveau d'expression (figure 14).

ScPro1 et SCB1 ont augmenté respectivement par 1,5 et 1,6 le niveau d'expression du gène codant pour le récepteur nucléaire PPARα à des doses de 100 μg. La combinaison ScPro1 + SCB1 (histogramme n°7) multiplie par 1,7 ce niveau d'expression (figure 15).

**EXEMPLE 5: Etude de l'influence des levures ScPro1 et SCB1 sur la colonisation au niveau de l'intestin de *Candida albicans* dans un modèle murin d'i**nflammation chimio-induite

L'étude vise à déterminer les effets de l'administration des levures ScPro1 et SCB1 de type probiotique sur la colonisation intestinale de la levure pathogène *Candida albicans* et son effet de potentialisation de l'inflammation dans un modèle murin de colite chimio-induite.

Les levures testées étaient sous forme sèche instantanée.

• *Conditions expérimentales :*

Les souris femelles de souche Balb/C sont âgées de 4 à 6 semaines. A partir de J0 jusqu'à J14, les animaux ont reçu du DSS (Dextran Sodium Sulfate) à 1,5% dans l'eau de boisson, pour chimio-induction de l'inflammation.

Trois expérimentations ont été réalisées.

Dans la première, à J-5, les souris ont été gavées par canule avec $5.10^7$ cellules de levure ScPro1 dans 200 μl de PBS (tampon phosphate). Cette opération a été renouvelée tous les jours pendant 19 jours. A J0, les souris ont été gavées par canule avec 5x10$^7$ cellules de levure de la souche de C. *albicans* SC5314 dans 200 μl de PBS.

Dans la deuxième, à J0, les souris ont été gavées par canule avec 5x10$^7$ cellules de levure de la souche de *C. albicans* SC5314 dans 200 μl de PBS. 4 jours après un lot de souris a fait l'objet d'un gavage par 5x10$^7$ cellules de levure ScPro1 dans 200 μl de PBS. Cette opération a été renouvelée tous les jours pendant 14 jours.

Dans la troisième, à J0, les souris ont été gavées par canule avec 5.10$^7$ cellules de levure de la souche de *C. albicans* SC5314 dans 200 μl de PBS. Une heure après un lot de souris a fait l'objet d'un gavage par 5.10$^7$ cellules de levure ScPro1 dans 200 μl de PBS. Cette dernière opération a été renouvelée tous les jours pendant 14 jours.

Les animaux (issus des expérimentations 1, 2 et 3) ont été suivis quotidiennement en ce qui concerne les points suivants :

- la consistance des selles, le saignement anal, leur masse corporelle (score clinique),
- des rétrocultures de 1 mg de selles homogénéisées dans 1 ml de PBS dont 10 μl ont été ensemencés sur milieu Candi-select ; après 24h de culture à 37°C les UFC de *C. albicans* (colorées en bleu) et de S. *cerevisiae* (colorées en vert) ont été dénombrées,
- les animaux ont été sacrifiés à l'issue des essais. Le sang a été prélevé immédiatement par ponction cardiaque, décanté à température ambiante, le sérum a été récupéré par centrifugation et stocké à -80°C ; le colon a été prélevé et réparti en 4 sections dont 3 ont été congelées et 1 a été placée dans le fixateur (PFA à 4%) pour étude histologique.

**• *Résultats* :**

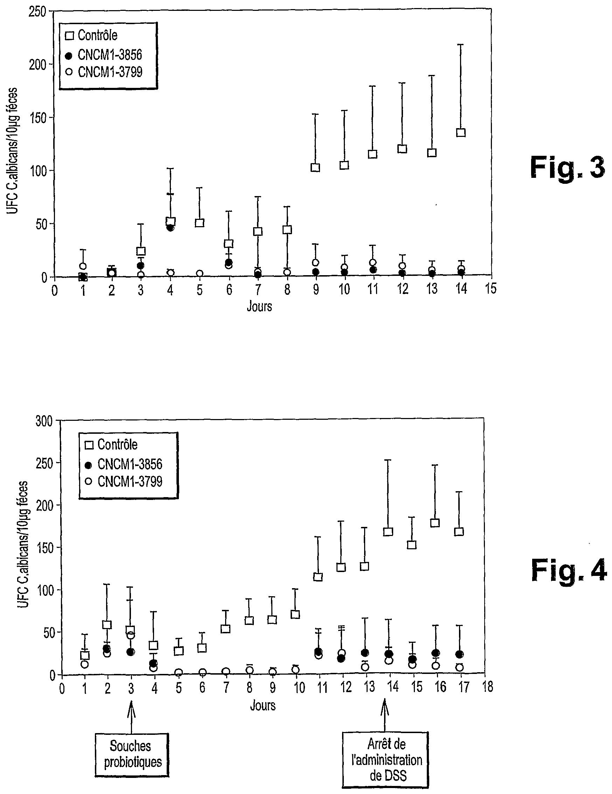

Comme on peut le voir sur la figure 3, dans la première expérimentation (test d'effet prophylactique), on a observé dans ce modèle de colite chimio-induite que l'administration de DSS augmente significativement la colonisation des muqeuses intestinales par *C. albicans* à partir du J4 (DSS+Ca). De manière très intéressante, on voit que l'administration de la levure probiotique ScPro1 durant 19 jours réduit significativement la colonisation de C. *albicans* induite par DSS.

Comme le montre la figure 4, dans la deuxième expérimentation (test de traitement), on a observé que l'administration de la levure probiotique ScPro1 ou SCB1 réduit la colonisation induite par DSS. De plus, les effets de la levure ScPro1 sont visibles même après l'arrêt du traitement par le DSS à J14.

**• *Conclusion* :**

Il en ressort que l'administration de la levure ScPro1 ou de la levure SCB1 réduit de façon significative la colonisation de *C. albicans,* et ce à la fois en condition prophylactique et en condition de traitement. Il est à noter que cet effet protecteur perdure même à l'arrêt du traitement.

**EXEMPLE 6 : Etude de l'effet inhibiteur de la Levure ScPro1 ou SCB1 ou dérivés sur le pouvoir d'adhésion et d'invasion de souches pathogènes de *E. coli* isolées de biopsies iléales de patients atteints de la Maladie de Crohn**

L'influence des levures vivantes ScPro1, SCB 1 et dérivés a été étudiée pour ses effets inhibiteurs sur le pouvoir d'adhésion et d'invasion de souches pathogènes de *E. coli* isolées de biopsies iléales de patients atteints de la maladie de Crohn.

Des souches de *E. coli* dénommées AIEC pour Adherent-Invasive *E. coli* isolées de biopsies iléales de patients atteints de maladie de Crohn (MC) sont capables d'adhérer et d'envahir les cellules épithéliales intestinales.

La souche de *E. coli* LF82, isolée d'une lésion iléale chronique chez un patient atteint de maladie de Crohn, possède toutes les caractéristiques d'un pathogène bactérien invasif. La caractérisation d'un phénotype d'adhésion-invasion de la souche LF82 et l'absence de déterminants génétiques d'invasion déjà décrits chez *E. coli, Shigella* et *Salmonella* a conduit à définir l'existence d'un nouveau groupe pathogène de *E. coli* pouvant être associé à la maladie de Crohn, désigné AIEC. Après phagocytose par des macrophages murins ou humains, la souche AIEC LF82 survit et se multiplie dans une large vacuole, tout en préservant l'intégrité de la cellule hôte. Suite à l'infection, les macrophages sécrètent un taux important de TNFα. La prévalence des souches AIEC est de 36,4% au niveau des lésions iléales de patients atteints de MC.

Le processus d'adhésion d'une bactérie à des cellules eucaryotes résulte d'une interaction spécifique entre un ligand présent à la surface de la bactérie, appelé adhésine, et un récepteur de nature protéique, glycoprotéique ou glycolipidique exprimé à la surface de la cellule épithéliale de l'hôte. En ce qui concerne les bactéries, il a été montré que l'adhésine FimH des pili de type 1 est impliquée dans l'adhésion des bactéries AIEC aux cellules épithéliales intestinales. L'adhésine bactérienne FimH reconnaît le récepteur entérocytaire CEACAM6 (dénommé également CD66c ou NCA) qui est une glycoprotéine riche en résidus mannose et anormalement surexprimée au niveau iléal chez 90% des patients atteints de MC.

***Conditions expérimentales :***

La souche AIEC LF82 caractérisée pour son pouvoir d'adhésion et d'invasion de cellules épithéliales intestinales en culture a été utilisée comme souche prototype.

Cette étude a été étendue à 10 souches AIEC isolées de patients atteints de MC afin de confirmer les résultats obtenus avec la souche AIEC LF82.

La souche d'E. coli DAEC (Diffuse Adherent *Escherichia Coli*) C1845, qui adhère aux cellules épithéliales via un mécanisme indépendant de mannose (adhesines Afa/Dr) est utilisé comme contrôle négatif.

**Essais d'agglutination**

Avec les levures ScPro1 et SCB1 vivantes, des essais d'agglutination quantitatifs ont été réalisés soit en présence des bactéries AIEC, soit en présence d'extraits purifiés de pili de type 1 préparés à partir de la souche AIEC LF82 selon le protocole décrit dans Boudeau et coll. (2001 *Mol Microbiol.* **39:**1272-84). Un index d'agglutination a été déterminé à l'aide d'une concentration fixe de levure et des concentrations variables de bactéries ou de pili de type 1 purifiés.

Dans le cas des fractions de levure de type mannoprotéique pour lesquelles on n'observe pas d'agglutination, une détermination de la capacité de liaison de pili de type 1 a été réalisée par technique ELISA.

Ces essais sont usuellement pratiqués en microplaques. Les fractions de levures sont fixées sur une microplaque. Diverses dilutions de pili de type 1 purifiés sont mises en contact avec les fractions de levures. Après lavages, les pili de type 1 sont révélés à l'aide d'anticorps anti-pili de type 1 obtenus chez le lapin (Boudeau et coll. 2001). Après lavages, des anticorps secondaires couplés à la peroxydase sont utilisés. La quantification est réalisée à l'aide du substrat de la peroxydase ($H_2O_2$) et d'un chromogène (tetraméthylbenzidine) et lecture de la microplaque à la densité optique de 450 nanomètres.

**Essais d'inhibition de l'interaction des bactéries AIEC avec le récepteur CEACAM6 exprimée à la surface de cellules épithéliales intestinales par la levure ScPro1 ou SCB1**

Cellules utilisées :

Pour les essais d'inhibition *in vitro (pré et co incubation),* des cellules épithéliales intestinales T84 non différenciées, exprimant fortement le récepteur CEACAM6, ont été retenues. Les cellules T84 ont été cultivées sous 5% de $CO_2$ à 37°C dans du milieu de base DMEM (Dulbecco's Modified Eagle's Medium) additionné de 50% de Ham-F12 (Life Technology) et de 10% sérum de veau foetal décomplémenté à la chaleur. A ce milieu ont été ajoutés 1% d'acides aminés non essentiels (Life Technology), 1% glutamine (Life Technology), 200 U/l pénicilline, 50 mg/l streptomycine, 0,25 mg/l amphotéricine B et 1% du mélange vitaminé X-100 pour milieu MEM (Minimum Essential Medium) (Life Technology). Les cellules ont été ensemencées à $4.10^5$ cellules par puits et par ml et ont été incubées 48h à 37°C, sous 5% de $CO_2$. Le tapis de cellules T84 a été ensuite lavé en PBS, puis 1 ml de milieu d'infection (DMEM/F12 + 10% de SVF) a été ajouté dans chaque puits. A partir d'une culture de la souche AIEC LF82 d'une nuit à 37°C en bouillon Luria-Bertani (LB), une suspension bactérienne à une $DO_{620}$ de 0,1 en PBS a été préparée. Les cellules T84 ont été infectées à une multiplicité d'infection (MOI) de 10 bactéries pour 1 cellule en ajoutant 25 $\mu$l de suspension bactérienne à $DO_{620}$ de 0,1 dans le milieu d'infection. Une plaque 24 puits a été incubée 3h à 37°C sous atmosphère enrichie en $CO_2$. L'adhésion et d'invasion résiduelle de bactéries ont été réalisées comme décrit ci-après.

Une expérimentation faisant appel à des cellules CHO-K1 qui n'expriment pas CEACAM6 et ces mêmes cellules génétiquement modifiées qui expriment de manière stable CEACAM6 (CHO-K1/CEACAM6) a été utilisée. Les cellules CHO-K1 ont été cultivées en milieu DMEM/F12, 5% de sérum de veau foetal, 1% de L-glutamine, 200U/l de pénicilline, 50 mg/l de streptomycine et 0,25 mg/l d'amphotéricine B. Les cellules CHO-K1/CEACAM6 ont été cultivées en milieu DMEM/F12, 5% de sérum de veau foetal, 1% de L-glutamine et 600$\mu$g/ml d'hygromycine. Les cellules ont été ensemencées en plaque 24 puits à $2.10^5$ cellules/puits. Après 7 à 8h d'incubation à 37°C, le milieu est remplacé par du milieu de culture neuf, additionné de 5mM de sodium de butyrate, afin d'induire l'expression de CEACAM6. Un western blot a été réalisé afin de contrôler l'expression de la protéine CEACAM6 par les cellules transfectées. Après 20-24h d'incubation à 37°C, les cellules ont été incubées avec des concentrations croissantes de la souche de levure ScPro 1 sèche instantanée pendant 1h (expérience de pré-incubation), puis elles ont été infectées à une MOI de 20 ($4.10^6$ bactéries/puits), afin de respecter le ratio bactéries/levures précédemment utilisé lors des expériences menées sur cellules T84. Après 3h d'incubation à 37°C, les bactéries adhérentes ont été dénombrées en absence ou en présence de levures comme décrit ci dessous.

Une autre expérimentation a utilisé des pièces opératoires issues de patients malades. Les entérocytes, issus de biopsies iléales de 3 patients atteints de maladie de Crohn, ont été lavés dans du PBS puis pré-incubés dans un tube eppendorf de 2 ml, dans 1 ml de milieu DMEM, 20% de sérum de veau foetal, en présence de 0, 1,25, 2,5 ou 5 mg/ml de la souche de levure ScPro1 sous forme sèche instantanée. Le tube a été placé en agitation par rotation pendant 15 min à 37°C, puis les entérocytes ont été infectés, en présence des levures, par 50 µl d'une culture en LB d'une nuit de la souche AIEC LF82. Une incubation de 3h avec agitation a été réalisée. Les entérocytes ont été lavés à 2 reprises dans du PBS, puis déposés entre lame et lamelle et observés en microscopie à contraste de phase. Des comptages de bactéries adhérentes à la bordure en brosse des entérocytes ont été effectués en absence ou en présence des levures. L'expérience a été également réalisée avec le mutant non pilié LF82-*delta fimH,* afin de déterminer le niveau d'adhésion basal des bactéries AIEC ne mettant pas en jeu la reconnaissance des pili de type 1 aux récepteurs CEACAM6. De même, des expériences d'inhibition d'adhésion ont été menées en présence d'anticorps anti-CEACAM6.

Protocole suivi pour mesurer l'adhésion et d'invasion résiduelle de bactéries à des cellules épithéliales intestinales T84

Le tapis cellulaire a été lavé à 4 reprises par 1 ml de PBS puis les cellules ont été lysées par 5 min d'incubation à température ambiante avec 500 µl de Triton X-100 1% en eau distillée. Les lysats ont été dilués puis étalés sur gélose LB-Agar pour déterminer le nombre de cfu, correspondant aux nombres de bactéries adhérentes.
Pour dénombrer les bactéries invasives, le tapis cellulaire a été lavé par du PBS suite aux 3h d'infection, puis a été incubé 1h avec 1 ml de milieu d'infection contenant 100 µg/ml de gentamicine, afin de détruire les bactéries extra-cellulaires. Les bactéries invasives ont été dénombrées après lyse des cellules, dilutions en série et étalement sur gélose LB-agar.
Les niveaux d'adhésion et d'invasion de la souche AIEC LF82 ont été analysés comparativement à des cellules infectées par la souche AIEC LF82 n'ayant subit aucun traitement par les levures ou dérivés de levure.
Tous les résultats seront exprimés selon le rapport R :

$$R = \text{Nombre de bactéries adhérentes ou invasives en présence de la levure ScPro1 / Nombre de bactéries adhérentes ou invasives sans traitement.}$$

Protocole 1 : modèle de co-incubation

Les cellules T84 et la suspension de bactéries ont été préparées comme décrit ci-dessus, lors des essais d'adhésion et d'invasion. Les levures ou les dérivés de levures ont été mis en suspension dans du PBS à une concentration déterminée, puis 25µl de cette suspension ont été ajoutés dans le milieu d'infection des cellules T84 (1 ml). Les cellules ont été ensuite immédiatement infectées à MOI = 10 avec la souche bactérienne. La suspension bactéries/levures incubées en présence des cellules a été homogénéisée, puis la plaque 24 puits a été incubée pendant 3h à 37°C. Les niveaux d'adhésion et d'invasion de la souche bactérienne ont été déterminés comme décrit ci-dessus, et ceci, en absence et en présence de levures ou d'extraits de levures lors de l'infection. Le rapport entre le niveau d'adhésion ou d'invasion bactérienne en absence de levure (100%) et le niveau d'adhésion ou d'invasion bactérienne en présence de levures représente le niveau d'adhésion ou d'invasion résiduelle des bactéries.

Protocole 2 : modèle de pré-incubation

Les cellules T84 et la suspension de bactéries ont été préparées comme décrit ci-dessus, lors des essais d'adhésion et d'invasion. La suspension de levures ou de dérivés de levures a été ajoutée dans le milieu d'infection (1 ml) des cellules T84 dans un volume de 25 µl. La suspension de levures a été homogénéisée puis la plaque de cellules 24 puits a été incubée 1h à 37°C. A la suite de cette incubation, les cellules T84 ont été infectées par la souche bactérienne, à MOI = 10, en présence des levures, et ceci pendant 3h à 37°C. Le dénombrement des bactéries adhérentes et invasives a été réalisé comme décrit ci-dessus, en présence ou en absence de levures, afin de déterminer un pourcentage d'adhésion ou d'invasion résiduelle, ou 100% représente l'adhésion ou l'invasion en absence de levure.

- Vérification de l'expression de CEACAM6.

Des marquages immunocytochimiques ont été réalisés sur chaque lot de cellules en culture pour vérifier la présence et estimer la quantité de CEACAM6 exprimée. Les cellules ont été cultivées sur des lamelles en verre stériles. Le tapis cellulaire a été lavé au PBS, puis fixé par du paraformaldéhyde 3% pH 7,4 pendant 10 minutes à température ambiante. Les cellules ont été incubées avec l'anticorps monoclonal anti-CEACAM6 (clone 9A7, Genovac) dilué au 1/100 dans du PBS-5% sérum de cheval, dans une atmosphère humide pendant une heure. Après lavage au PBS, les cellules ont été mises en contact avec un anticorps secondaire couplé à un fluorochrome (FITC-anti-souris, Zymed) dilué au 1/500 dans du PBS-5% sérum de cheval, pendant 1 heure en atmosphère humide. Les lamelles de verre ont été fixées sur lame au Moewiol, puis visualisées avec un microscope à fluorescence.

- Contrôle d'absence de cytotoxicité cellulaire.

L'absence de cytotoxicité cellulaire induite par les différentes doses de levure a été testée par dosage de lactate déshydrogénase (LDH) dans le milieu d'incubation levures/cellules ou FDL/cellules (Glasser et coll, 2001).

*Résultats :*

Essais d'agglutination avec LF82

Les titres d'agglutination obtenus avec LF82 en présence de la levure ScPro1 ou SCB 1 en culture (= forme fraîche) ou sous forme sèche (séchage sèche instantanée ou lyophilisé) sont résumés sur le tableau suivant qui est le résultat de 3 à 5 manipulations indépendantes :

| | | Titre d'agglutination | | |
|---|---|---|---|---|
| Levure | Forme | Moyenne | Titre mini | Titre max |
| ScPro1 | culture fraîche | 1/7 | 1/3 | 1/12 |
| ScPro1 | sèche instantanée | 1/58 | 1/20 | 1/96 |
| ScPro1 | Sèche lyophilisée | 1/43 | 1/24 | 1/64 |
| SCB1 | Culture fraîche | 1/28 | 1/12 | 1/40 |
| SCB1 | Sèche instantanée | 1/16 | 1/12 | 1/20 |
| SCB1 | sèche lyophilisée | 1/19 | 1/16 | 1/24 |

De manière absolue, de bons résultats d'agglutination avec LF82 sont obtenus avec la levure sèche ScPro1 sèche instantanée et la levure SCB1 sèche instantanée.
Pour ces levures, on a montré l'importance de l'impact favorable du procédé et notamment du procédé de séchage sur son potentiel d'agglutination.

Essais d'agglutination avec les pili purifiés

Les titres d'agglutination obtenus avec les pili purifiés en présence de la levure ScPro1 en culture (= forme fraîche) ou sous forme sèche instantanée et de la levure SCB1 (sèche) sont résumés sur le tableau suivant :

| | | Titre d'agglutination | | |
|---|---|---|---|---|
| Levure | Forme | exp 1 | exp 2 | exp 3 |
| ScPro1 | frais - levure pressée | 1/300 | 1/300 | 1/400 |
| ScPro1 | Sèche instantanée | 1/600 | 1/600 | 1/300 |
| SCB1 | sèche lyophilisée | 1/300 | 1/300 | 1/200 |

Cette expérience confirme qu'une interaction pili - levure est bien nécessaire pour l'agglutination. Les pili ayant pour propriété de reconnaître des structures mannose, ce sont ces dernières qui sont reconnues sur les levures et qui participent au phénomène d'agglutination observé. Les meilleurs résultats sont obtenus avec la levure ScPro1 sèche instantanée.

Résultat de l'essais de détermination de la capacité de liaison de pili de type 1 sur des fractions mannoprotéiques de levure ScPro1

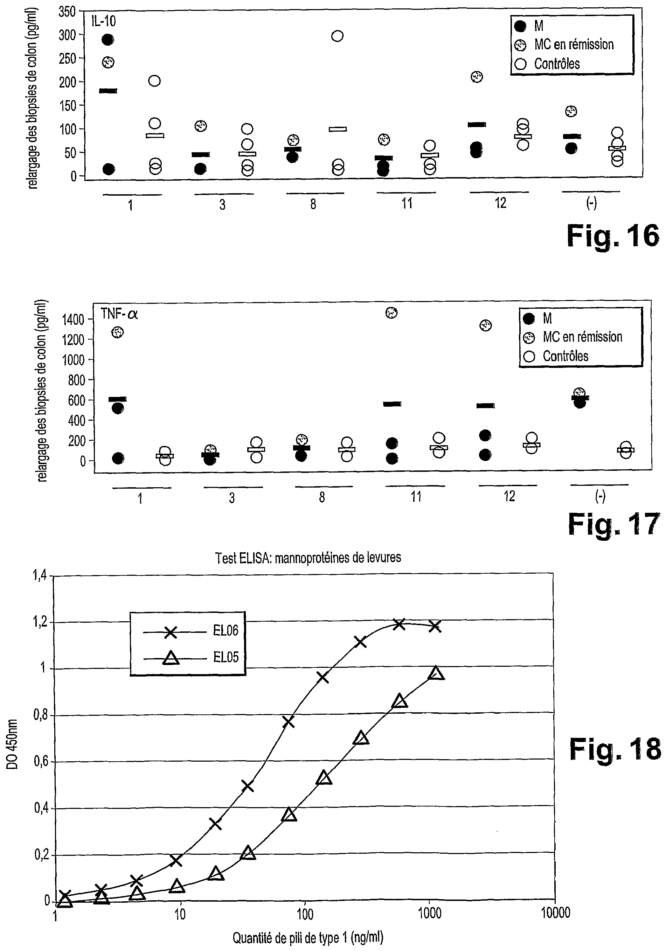

La figure 18 montre clairement que les pili de type 1 purifiés à partir de la souche AIEC LF82 se fixent spécifiquement sur les mannoprotéines de levure. On remarque que la méthode de préparation (thermique ou enzymatique) de ces mannoprotéines (EL05 et EL06) influe un peu sur la constante d'affinité des pili.

Résultats d'inhibition de l'interaction de bactéries AIEC avec le récepteur CEACAM6 exprimé à la surface de cellules épithéliales

**1/ Résultats du criblage d'échantillons de levures ou de dérivés de levures pour leur pouvoir d'inhibition de l'adhésion et de l'invasion de la souche AIEC LF82 aux cellules épithéliales intestinales T84 dans un modèle de co-incubation.**

Les levures ScPro1 sèche instantanée ($3,09.10^7$ levures/mg), ScPro1 sèche ($1,86.10^7$ levures/mg) et ScB1 sèche instantanée ($5,83.10^7$ levures/mg), ainsi que les mannoprotéines de levure EL05 (forme sèche) ont été étudiées.
A titre de comparaison, l'Ultra-levure® (Biocodex à $2,054.10^7$ levures/mg) a été ajoutée.

**Comparaison du pouvoir inhibiteur des levures à nombres de levures identiques dans le modèle de co-incubation**

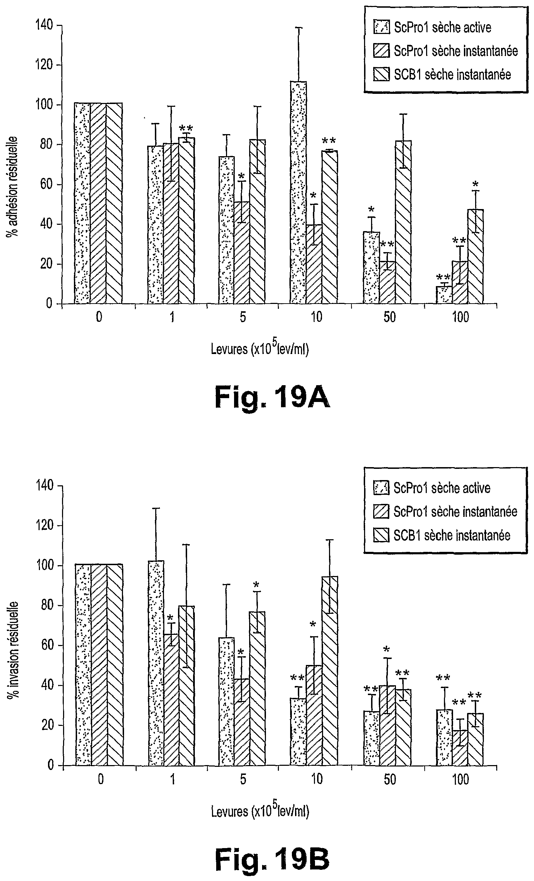

Après un lavage avec du PBS et une centrifugation de 15 min à 7500 rpm, les échantillons de levures ont été remis en suspension à une concentration de $4.10^8$ levures/ml dans du PBS. Des dilutions des levures dans du PBS ont été effectuées : 1/2, 1/10ème, 1/20ème et 1/100ème.
Trois expériences indépendantes ont été réalisées en utilisant le protocole 1. Les résultats présentés sur les figures 19A et 19B (Adhésion résiduelle et Invasion résiduelle) sont présentés sous forme de moyennes des taux d'adhésion et d'invasion résiduelles et les barres d'erreur correspondent à l'erreur standard à la moyenne.
Les figures 19A et 19B permettent d'obtenir les résultats suivants :

Adhésion :

- Les levures ScPro1 sèche instantanée et ScPro1 sèche inhibent fortement l'adhésion de la souche LF82 aux cellules T84 de manière dose-dépendante. L'inhibition est significative dès $5.10^5$ levures/ml pour la levure ScPro1 sèche instantanée, contre $5.10^6$ levures/ml pour la levure ScPro1 sèche.
- La levure ScB1 sèche instantanée inhibe moins fortement l'adhésion que les 2 autres échantillons de levures, avec 45,7% d'adhésion résiduelle à $1.10^7$ levures/ml, contre 18,7% et 8% d'adhésion résiduelle respectivement pour les souches ScPro1 sèche instantanée et ScPro1 sèche.

Invasion :

- La levure ScPro1 sèche instantanée inhibe significativement l'invasion des cellules T84 par la souche AIEC LF82 dès $1.10^5$ levures/ml. A $1.10^7$ levures/ml, le taux d'invasion résiduel est de 16,3%.
- Pour les levures ScPro1 sèche et SCB1 sèche instantanée, l'effet inhibiteur est plus tardif, respectivement à partir de $1.10^6$ levures/ml et $5.10^6$ levures/ml.

**Essais d'inhibition avec des mannoprotéines EL05 dans le modèle de co-incubation**

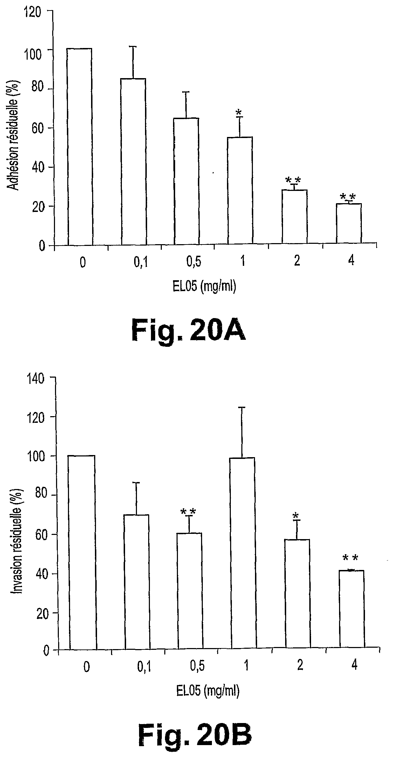

Des mannoprotéines de levures EL05 ont été mises en suspension dans du PBS à une concentration de 160 mg/ml. Des dilutions en séries ont été réalisées dans du PBS : 1/2, 1/4, 1/8 et 1/40 et 25 µl de chaque suspension de mannoprotéines ont été ajoutés au milieu d'infection en utilisant le protocole 1.
Trois expériences indépendantes ont été réalisées. Les résultats représentés par les figures 20A et 20B (Adhésion résiduelle et Invasion résiduelle) sont présentés sous forme de moyennes des taux d'adhésion résiduelles et les barres d'erreur correspondent à l'erreur standard à la moyenne.
Ces figures montrent que les mannoprotéines de la levure EL05 ont la capacité d'inhiber l'adhésion et l'invasion de la souche AIEC LF82 aux cellules T84 de manière dose-dépendante en modèle de co-incubation.

**2/ Criblage d'échantillons de levures ou de produits de levures pour leur pouvoir d'inhibition de l'adhésion et de l'invasion de la souche AIEC LF82 aux cellules épithéliales intestinales T84 dans un modèle de pré-incubation.**

Les mêmes échantillons de levure et fractions que ceux qui ont été utilisés pour le modèle co-incubation ont servi dans ce modèle de pré-incubation.

**Comparaison du pouvoir inhibiteur des levures à nombres de levures identiques dans le modèle de pré-incubation**

Après un lavage avec du PBS et une centrifugation de 15 min à 7500 t/min, les échantillons de levures ont été remis en suspension à une concentration de $4.10^8$ levures/ml en PBS. Des dilutions des levures en PBS ont été effectuées : 1/2, 1/10ème, 1/20ème et 1/100ème. Trois expériences indépendantes ont été réalisées en utilisant le protocole 2.

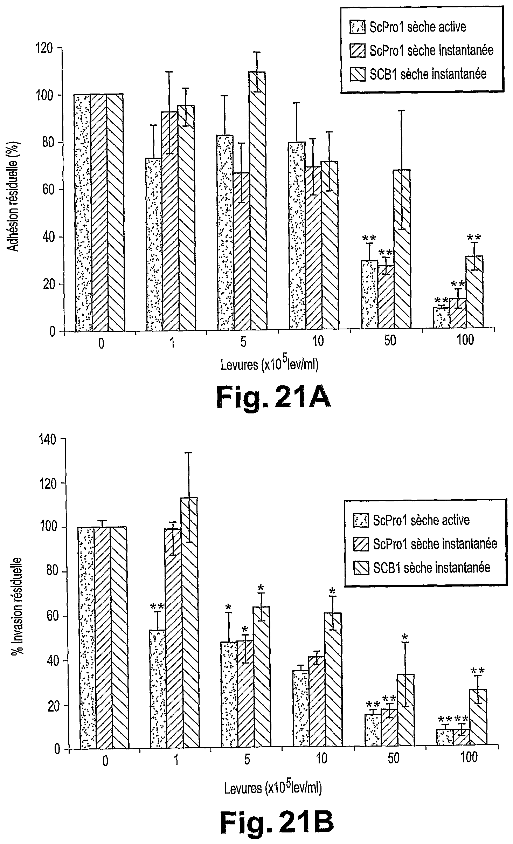

Les résultats représentés par les figures 21A et 21B (Adhésion résiduelle et Invasion résiduelle) sont présentés sous forme de moyennes des taux d'adhésion et d'invasion résiduelles et les barres d'erreur correspondent à l'erreur standard à la moyenne.

Le prétraitement des cellules T84 avec les levures permet d'obtenir une inhibition significative de l'adhésion de la souche LF82 dès la dose de $5.10^6$ levures/ml pour la souche ScPro1 sèche instantanée et la souche scPro1 sèche. Toutefois, à cette dose, aucune inhibition significative n'a été observée avec la levure ScB1 sèche instantanée.

Le prétraitement des cellules T84 avec les levures permet d'obtenir une inhibition de l'invasion de la souche LF82 dès la dose de $1.10^5$ levures/ml pour la souche de levures ScPro1 sèche.

A partir de la dose de $5.10^5$ levures/ml, les 3 échantillons de levures induisent une diminution significative de l'invasion de la souche LF82.

**Essais d'inhibition avec les mannoprotéines EL05 dans le modèle de pré-incubation**

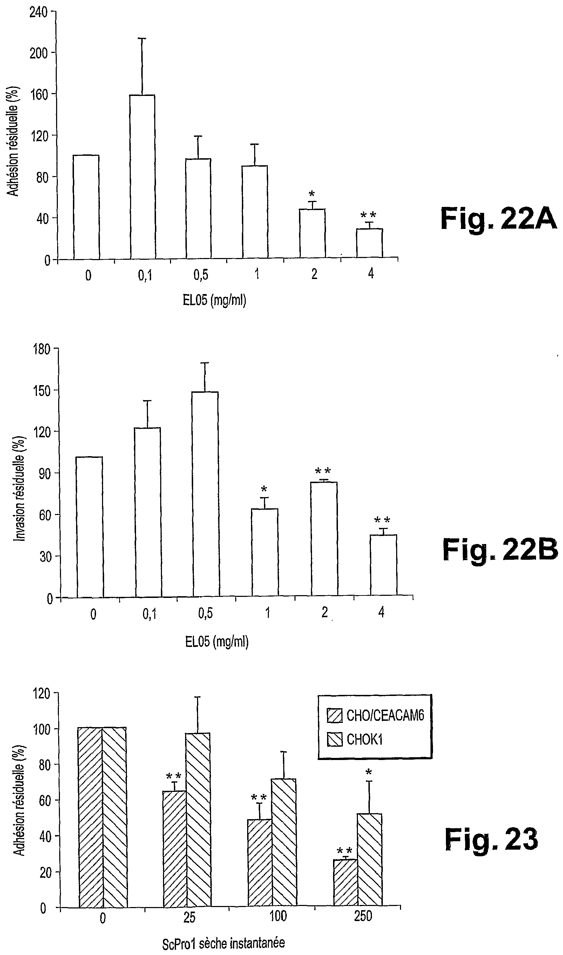

Les mannoprotéines de levures EL05 ont été mises en suspension en PBS à une concentration de 160 mg/ml. Des dilutions en séries ont été réalisées en PBS : 1/2, 1/4, 1/8 et 1/40 et 25 $\mu$l de chaque suspension de mannoprotéines ont été ajoutés au milieu d'infection en utilisant le protocole 2. Trois expériences indépendantes ont été réalisées. Les résultats des figures 22A et 22B (Adhésion résiduelle et Invasion résiduelle) sont présentés sous forme de moyennes des taux d'adhésion résiduelles et les barres d'erreur correspondent à l'erreur standard à la moyenne.

Les mannoprotéines EL05 permettent d'inhiber de façon dose-dépendante l'adhésion et l'invasion de la souche LF82 à partir de la concentration de 2 mg/ml.

**Résultats du test d'inhibition par les levures de l'adhésion de la souche AIEC LF82 à des CHO-K1 exprimant ou non le récepteur CEACAM6 dans le modèle de pré-incubation**

Cinq expériences indépendantes ont été réalisées en utilisant le protocole 2 mentionné précédemment.

La figure 23 montre qu'une inhibition significative de l'adhésion de la souche AIEC LF82 est observée avec les cellules CHO/CEACAM6 dès la pré-incubation avec 25 $\mu$g/ml de levures. Un effet inhibiteur dose-dépendant est observé avec ces cellules.

Une adhésion de la souche AIEC LF82 est également observée aux cellules CHO-K1, certainement due à l'expression de protéines mannosylées exprimées à la surface de ces cellules. Toutefois, la pré-incubation de la souche LF82 avec la levure ScPro1 sèche instantanée ne permet pas d'inhiber de façon très significative l'adhésion aux cellules non transfectées.

Ceci atteste donc que la levure interfère dans l'adhésion de la souche LF82 aux récepteurs CEACAM6 exprimés par les cellules.

**Résultats d'inhibition de l'adhésion de la souche AIEC LF82 au niveau de la bordure en brosse d'entérocytes de patients atteints de maladie de Crohn dans le modèle de pré-incubation**

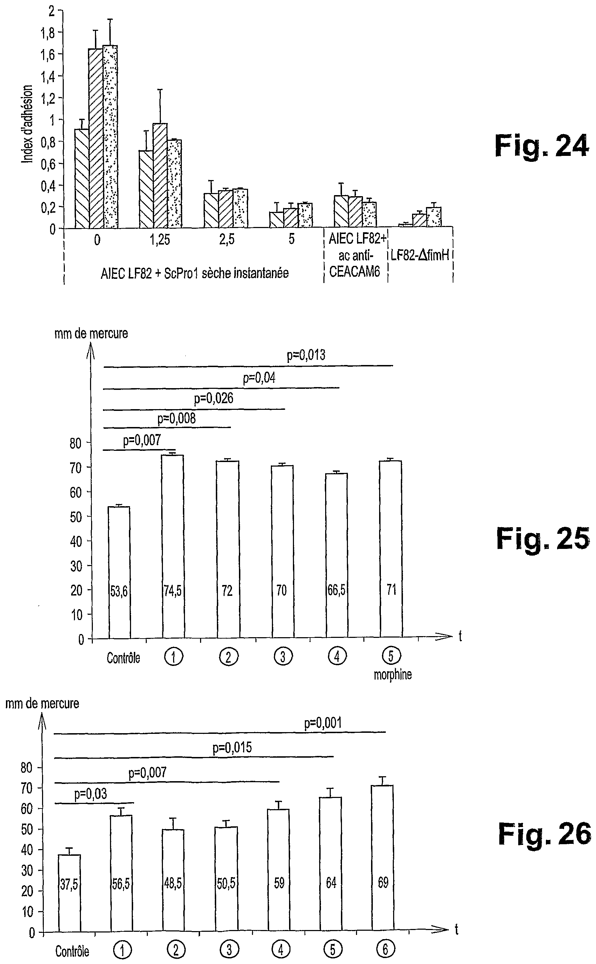

La figure 24 montre les indices d'adhésion moyens obtenus au cours de l'expérimentation et qui ont été calculés en présence ou en absence de concentrations croissantes de levures ScPro1 sous forme sèche instantanée (mg/ml) ou en présence d'anticorps anti-CEACAM6. D'après les résultats de cette figure, on constate une diminution significative et dose-dépendante de la souche AIEC LF82 à la bordure en brosse des entérocytes de patients lorsque l'on est en présence de la souche de levure ScPro1 sèche instantanée. A la dose de 5 mg/ml de levures, l'adhésion résiduelle de la souche AIEC LF82 est similaire à celle observée en présence d'anticorps anti-CEACAM6 ou à celle observée pour un mutant dépourvu de pili de type 1.

*Conclusion :*

Il ressort de cette étude que :

- Les levures ScPro1 et SCB1, en particulier sous forme sèche instantanée, présentent un fort pouvoir d'agglutination de la souche LF82.
- Les levures ScPro1 et SCB1 sont capables d'inhiber in vitro l'adhésion et l'invasion de cellules épithéliales humaines (T84, entérocytes de biopsie iléale) et de cellules CHO exprimant le récepteur CEACAM6 humain par des *E. coli* de manière dose dépendante.
- Les mannoprotéines sont capables d'inhiber in vitro l'adhésion et l'invasion de cellules épithéliales humaines (T84, entérocytes de biopsie iléale) et de cellules CHO exprimant le récepteur CEACAM6 humain par des *E. coli* de manière dose dépendante.
- In vitro, la levure ScPro1 est capable, à des fortes concentrations, de protéger partiellement, à environ 80% les cellules de l'infection bactérienne.

**EXEMPLE 7 : Etude du rôle régulateur des levures ScPro1, SCB1 et des dérivés de levure sur l'expression des gènes codant pour IL-10 et PPAR$\alpha$ chez des cellules épithéliales intestinales humaines cultivées *in vitro***

On a étudié le caractère probiotique des levures ScPro1 et SCB1, prises seules ou en combinaison, et/ou de fractions de levure, et leur faculté d'inhiber le déclenchement des inflammations par interaction avec certains récepteurs intestinaux.

Essais in vitro

On a notamment étudié les effets de la levure et des dérivés de levure selon l'invention sur différents récepteurs de cellules épithéliales intestinales par une analyse *in vitro* sur deux lignées cellulaires de cancer du côlon CaCo-2 (ATCC HTB-37) et HT-29 (ATCC HTB-38).

Pour cela, on a effectué une analyse transcriptionnelle par extraction de l'ARN, selon la méthode suivante.

Les cellules sont lysées dans du Trizol. Sur la fraction soluble, on réalise ensuite une étape de désoxyribonucléase en ajoutant 200$\mu$l d'une solution contenant 10 U d'inhibiteur de ribonucléase et 10U de désoxyribonucléase.

10 $\mu$g d'ARN ont été rétrotranscrits en présence de 200 U de transcriptase inverse, de dithiotréitol, d'oligo-dT15 et de désoxyribonucléotides.

Les ADNc sont amplifiés par la technique connue de réaction en chaîne de polymérisation (PCR Polymerase Chain Reaction en anglais) en même temps qu'un compétiteur en utilisant des amorces sens et anti-sens spécifiques, notamment des gènes suivants : IL-10 et PPAR$\alpha$.

Après 40 cycles d'amplification, réalisés en présence de 1,25 U d'Ampli Taq Gold 5000 et migration des différents échantillons sur gel d'agarose 3%, l'intensité des bandes est déterminée par un analyseur d'image.

Les résultats sont exprimés en nombre de molécules d'ARNm pour $10^5$ molécules d'un étalon interne : la $\beta$-actine.

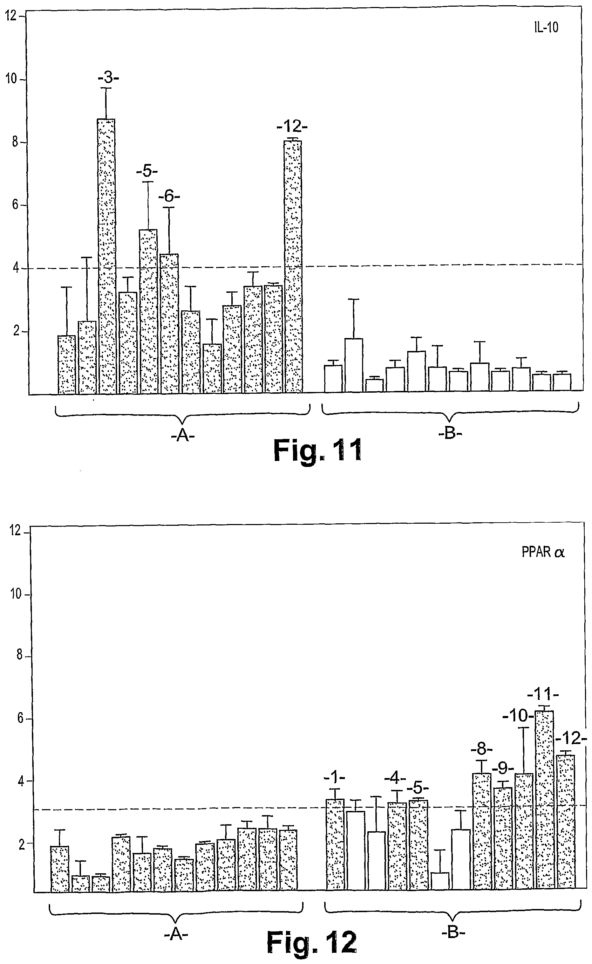

Les résultats, rassemblés sur la figure 11, comprennent les valeurs d'expression d'ARNm une heure après (sous la référence A), et 3 heures après (sous la référence B) la mise en contact des levures ou dérivés avec les cellules épithéliales intestinales du gène codant pour la protéine anti-inflammatoire IL-10.

Sur cette figure 11, les références suivantes désignent les levures/dérivés de levure expérimentés, qui ont montré un niveau d'expression précoce supérieur à 4 fois le signal de référence :

3 désigne une *levure Saccharomyces cerevisiae,*
5 désigne la levure ScPro1 de l'invention,
6 désigne un extrait de levure *Saccharomyces cerevisiae,* et
12 désigne une fraction d'ARN de levure *Saccharomyces cerevisiae.*

Ces résultats montrent bien que la levure et les dérivés de levure *Saccharomyces cerevisiae,* selon l'invention, induisent l'expression de manière précoce, après une heure, du gène codant pour la cytokine anti-inflammatoire IL-10.

En effet, par rapport au témoin non traité, l'expression en ARNm pour la levure et les dérivés selon l'invention est supérieure à 4 sur l'axe des ordonnées, valeur qui correspond déjà à un excellent signal d'expression précoce.

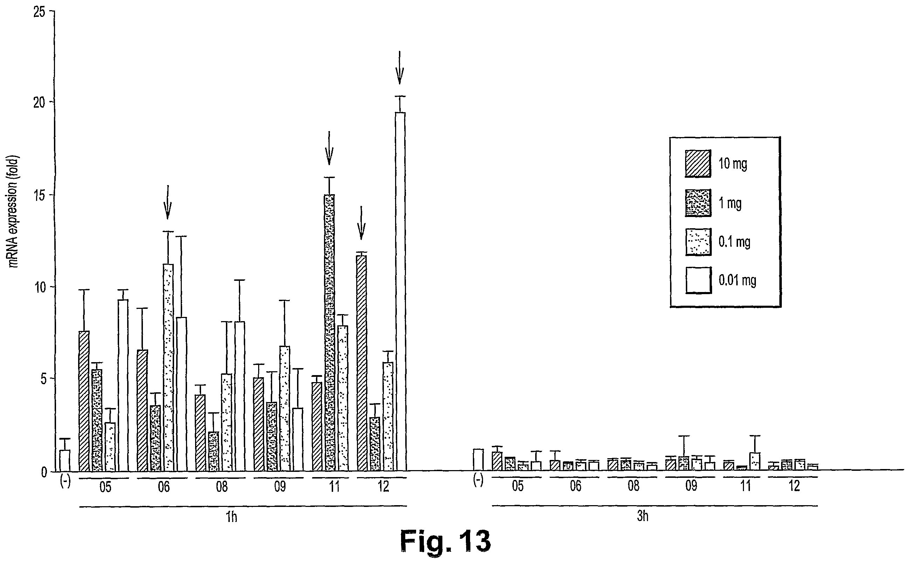

D'autres résultats sont présentés sur la figure 13. Cette figure montre la modulation, en fonction des quantités de dérivés de levure apportées, de l'expression de l'ARNm du gène codant pour la protéine IL-10.

Sur cette figure 13, l'expression a été mesurée après une heure (1h) et après trois heures (3h). On peut voir, l'expression des levures et extraits de levure *Saccharomyces cerevisiae* selon l'invention, désignés par les références suivantes :

5 désigne la levure ScPro1 selon l'invention,
6 désigne un extrait de levure *Saccharomyces cerevisiae,*
8 désigne un β-glucane pariétal de *Saccharomyces cerevisiae,*
9 désigne une mannoprotéine pariétale de levure *Saccharomyces cerevisiae,*
11 désigne une fraction d'ADN de levure *Saccharomyces cerevisiae,* et
12 désigne une fraction d'ARN de levure *Saccharomyces cerevisiae.*

L'expression a été mesurée avec des concentrations en levure et /ou dérivé différentes.

Plus la couleur des colonnes sur la figure 13 est foncée, plus la concentration en levure /dérivé est élevée.

Les résultats de cette figure 13 montrent que les extraits de levure *Saccharomyces cerevisiae* selon l'invention induisent de façon précoce les ARNm de la cytokine anti-inflammatoire (IL-10).

Les résultats rassemblés sur la figure 12 comprennent les valeurs d'expression d'ARNm une heure après (sous la référence A), et 3 heures après (sous la référence B) la mise au contact des levures ou dérivés avec les cellules épithéliales intestinales, du gène codant pour le récepteur nucléaire PPARα.

Sur cette figure 12, les références suivantes désignent les levures/dérivés de levure testés, qui ont montré un niveau d'expression tardive supérieur à 3 fois le signal de référence :

1 désigne la levure *Saccharomyces cerevisiae* ScPro1 selon l'invention sous une forme sèche active,
4 désigne une levure *Saccharomyces cerevisiae,*
5 désigne la levure Saccharomyces cerevisiae ScPro1 selon l'invention sous une forme sèche active,
8 désigne une fraction de parois de levure *Saccharomyces cerevisiae,*
9 désigne une fraction de β-glucanes pariétaux de levure *Saccharomyces cerevisiae,*
10 désigne une fraction de mannoprotéines pariétales de levure *Saccharomyces cerevisiae,*
11 désigne une fraction d'ADN de levure *Saccharomyces cerevisiae,* et
12 désigne une fraction d'ARN de levure *Saccharomyces cerevisiae.*

Ces résultats montrent bien que la levure et les dérivés de levure *Saccharomyces cerevisiae,* selon l'invention, induisent l'expression de manière tardive, après trois heures, du gène codant pour le récepteur nucléaire PPARα.

En effet, l'expression en ARNm pour la levure et les dérivés selon l'invention est supérieure à 3 sur l'axe des ordonnées, valeur qui correspond déjà à un excellent signal d'expression tardive.

**EXEMPLE 8 : Etude *ex vivo* du rôle régulateur des levures et des dérivés de levure sur l'expression des gènes codant pour IL-10 et TNF-α chez des cellules épithéliales intestinales humaines isolées de biopsies de patients atteints de la maladie de Crohn**

L'influence des levures et /ou des dérivés de levure sur la sécrétion des cytokines IL-10 (anti-inflammatoire) et TNF-α (pro-inflammatoire) a été étudié ex-vivo sur des biopsies de patients atteints de la maladie de Crohn ou non.

Des biopsies intestinales ont été prélevées sur des patients atteints de la maladie de Crohn ou non, puis placées 24h dans du milieu HBSS-CMF supplémenté en pénicilline et en streptomycine, à 37°C dans une atmosphère contenant 5 % de $CO_2$. Après lavage, les biopsies ont été mises en contact avec les levures ou les dérivés de levure pendant 4 heures dans du milieu RPMI 1640. Le surnageant a été récupéré pour analyse par ELISA. Les biopsies ont été ensuite lysées pour permettre une extraction soit d'ARNm soit de protéines totales.

La confirmation de la sécrétion des cytokines a été réalisée par analyse immunologique des surnageants de cultures de cellules et des protéines extraites par ELISA. Les protéines dénaturées pendant 5 minutes à 95°C dans un tampon de dépôt (vol/vol ; 75 mM Tris pH 6,8 ; 5 % glycérol ; 0,25 % bleu de bromophénol; 2 % SDS, 5% β-mercaptoéthanol ont été déposées (50 μg) et résolues sur un gel de polyacrylamide à 10 %. Après migration, les protéines ont été transférées sur une membrane de PVDF (Hybond-P, Amersham Pharmacia Biotech, Orsay, France) par électrotransfert semi-sec (Hoefer TE77, Amersham Pharmacia Biotech, Orsay, France) pendant 1 heure à 16 V. PPARα et IL-10 ont été révélés grâce à des antiséra polyclonaux de lapin anti-PPARα humain et anti-IL-10 humains dilués au 1/500e et quantifiés par chimiluminescence (E.C.L Amersham Pharmacia Biotech, Orsay, France) sur un film Biomax-MR (Kodak) à l'aide du logiciel Gel Analyst (CLARA VISION, Paris, France).

Les figures 16 et 17 montrent les résultats obtenus, respectivement, pour IL-10 et TNF-α. L'axe des ordonnées indique les quantités de cytokines mesurées en pg/ml. Chaque point correspond à la mesure d'une biopsie sur un patient atteint de la maladie de Crohn en phase aiguë (rond noir), sur un patient en rémission de maladie de Crohn (rond gris) et sur des patients sains (rond blanc). Une barre indique la moyenne des mesures.

Dans ces figures :

- 1 désigne la levure *Saccharomyces cerevisiae* ScPro1 selon l'invention sous une forme sèche active,
- 3 désigne une levure *Saccharomyces cerevisiae,*
- 8 désigne une fraction de parois de levure *Saccharomyces cerevisiae,*
- 11 désigne une fraction d'ADN de levure *Saccharomyces cerevisiae,* et
- 12 désigne une fraction d'ARN de levure *Saccharomyces cerevisiae.*

Sur la figure 16, la levure ScPro1 selon l'invention multiplie par 2 la sécrétion de la cytokine anti-inflammatoire IL-10 par les cellules épithéliales des patients atteints de la maladie de Crohn ou en rémission par rapport aux patients sains et au témoin négatif (-) correspondant aux mesures effectuées en présence d'eau physiologique seule.

La figure 17 montre que la levure ScPro1 selon l'invention n'entraîne pas d'augmentation de la sécrétion de la cytokine pro-inflammatoire TNF-$\alpha$ par les cellules intestinales isolées de biopsies de patients atteints de la maladie de Crohn ou en rémission. ScPro1, ni aucune des autres levures ou dérivés de levure testés n'induisent aucune sécrétion de TNF-$\alpha$.

## EXEMPLE 9 : Etude des propriétés analgésiques des levures et des dérivés de levure dans un modèle murin de distension colorectale

### Chez le rat sain

### 1 / Matériels et Méthodes

Des rats mâles Sprague Dawley (Charles River, l'Arbresle, France) pesant entre 175 et 200 g sont utilisés au cours de ces essais. Les rats sont acclimatés aux conditions de l'animalerie une semaine avant l'expérimentation. Ils sont maintenus à raison de cinq animaux par cage, avec eau et nourriture ad libitum. Tous les essais sont menés selon les recommandations du Committee for Research and Ethical Issues de l'International Association for the Study of Pain [6]. Des précautions sont prises pour éviter ou minimiser l'inconfort des animaux.

### 2/ Evaluation de la sensibilité du colon

La nociception des animaux est estimée en mesurant la pression intracolonique nécessaire pour induire une réponse comportementale. Cette pression est générée par distension colorectale au moyen du gonflement d'un ballon introduit dans le colon.

La réponse comportementale est caractérisée par une élévation de la partie arrière du corps de l'animal et une contraction abdominale clairement visible correspondant à de sévères contractions [7-9]. Les rats sont anesthésiés avec un anesthésiant volatile (2% isoflurane) et le ballon (préparé selon la procédure décrite par Bourdu [8] est inséré par voie intrarectale de manière la moins invasive possible, à 7 cm de l'anus. Le cathéter est attaché à la base de la queue par un ruban adhésif. Après 5 minutes, les rats sont placés au milieu d'une boite en Plexiglas et le cathéter est connecté à un barostat électronique (Distender Series IIRTM, G & J Electronics). Une pression croissante est appliquée continuellement jusqu'au déclenchement du réflexe de douleur ou jusqu'à ce que la pression limite de 80 mm de mercure soit atteinte.

### 3/ Composés administrés

Les levures ont été administrées par gavage une fois par jour pendant 15 jours.

La morphine injectée en intrapéritonéal à une dose de 1mg/kg est utilisée comme témoin positif, 30 min avant la distension colorectale.

8 groupes de rats ont été étudiés :

- 10 rats de contrôles recevant du PBS,
- 10 rats recevant la ScPro1 sèche instantanée (100$\mu$g/j), (référence 1)
- 10 rats recevant la souche ScPro1 sèche active (100$\mu$g/j), (référence 2)
- 10 rats recevant la souche SCB1 (100$\mu$g/j), (référence 3)
- 10 rats recevant les souches ScPro1 sèche instantanée (50$\mu$g/j) + SCB1 sèche instantanée (50$\mu$g/j), (référence 4)
- 10 rats recevant 1 injection de morphine (1mg/kg, 30 min avant la distension) (référence 5).

**4/ Résultats**

La figure 26 montre d'une part que la levure ScPro1 sous sa forme sèche instantanée, administrée seule (référence 1) ou en combinaison avec la souche SCB1 (référence 4), et d'autre part sous sa forme sèche active (référence 2), augmente le seuil de perception de la douleur, réduisant ainsi significativement la perception de la douleur viscérale comparée aux rats auxquels rien n'a été administré.

Les résultats suivants sont donnés en mm de mercure en comparaison avec le contrôle:

- $74,5 \pm 3,07$ vs $53,6 \pm 3.9$, p=0,07 pour la souche ScPro 1 sèche instantanée -(référence 1),
- $66,5 \pm 3,36$ vs $53,6 \pm 3.9$, p= 0,04 pour la combinaison des souches ScPro1 et SCB1 sèche instantanée - (référence 4),
- $72 \pm 2,59$ vs $53,6 \pm 3.9$, p<0.01 pour la souche ScPro1 sèche instantanée- (référence 2).

D'autre part, cet effet est comparable à celui induit par la morphine - référence 5-utilisée à la dose de 1mg/kg avec un seuil de douleur de $72 \pm 2,59$ mm de mercure.

La souche SCB1 sèche instantanée induit également un effet analgésique chez les rats sains $70 \pm 3,16$ mm de mercure p= 0,026.

**Chez le rat avant une hypersensibilité viscérale**

**1 / Matériels et Méthodes**

Des rats femelles Sprague Dawley (Charles River, l'Arbresle, France) pesant entre environ 175 et 200 g ont été utilisés. Les rats ont été acclimatés aux conditions du laboratoire une semaine avant l'expérimentation. Ils ont été maintenus à raison de cinq animaux par cage avec eau et nourriture ad libitum. Tous les essais ont été menés selon les recommandations du Committee for Research and Ethical Issues de l'International Association for the Study of Pain [6]. De grandes précautions ont été prises en qui concerne les conditions de vie afin d'éviter ou de minimiser l'inconfort des animaux.

**2 / Induction de l'hypersensibilité du colon par lavements au butyrate**

Pour chaque lavement, un cathéter (2-mm Fogarty) a été introduit dans le colon à 7 cm de l'anus et les animaux ont reçu, 2 fois par jour pendant 3 jours, 1 ml d'une solution 200 mM de butyrate de sodium de pH neutre (pH 6,9). Les animaux « sains » ont reçu une solution saline.

**3/ Traitement des animaux avec les levures selon l'invention**

10 groupes d'animaux présentant une hypersensibilité viscérale ont été utilisés (n=10 par groupe). Les animaux traités ont reçu 100µg de levures en gavage gastrique, une fois par jour pendant 15 jours. Les animaux témoins ont reçu du PBS selon le même protocole que précédemment. Les levures ont été mises en suspension dans une solution de PBS. Les instillations de butyrate ou de solutions salines commencent 7 jours après le premier gavage, pendant 3 jours. L'hypersensibilité colique a été mesurée 14 jours après le début du traitement par voie orale, soit 7 jours après les instillations coliques.

**4/ Groupes d'animaux étudiés**

Sept groupes de rats ont été étudiés. Les références numériques correspondent à la figure 26.

10 rats recevant du PBS (témoin),
10 rats recevant de la levure sèche ScPro 1 sèche instantanée (100 µg/jour) - (référence 1),
10 rats recevant de la levure sèche ScPro 1 sèche active (100 µg/jour) - (référence 2),
10 rats recevant de la levure SCB1 sèche instantanée (100 µg/jour) - (référence 3),
10 rats recevant ScPro 1 sèche instantanée (50 µg/jour) et SCB1 sèche instantanée (50 µg/jour) -(référence 4),
10 rats recevant de la morphine - (référence 5),
10 rats recevant des fibrates - (référence 6).

**5 / Résultats**

On peut tout d'abord noter que pour les rats témoins, le seuil de douleur dans les essais sur rats hypersensibilisés est inférieur à celui des rats sains.

La levure ScPro 1 sous forme sèche instantanée, prise seule ou mise en combinaison avec la levure SCB1 montrent des effets analgésiques intéressants dans ce modèle.

Les valeurs numériques sont les suivantes :

| Références | mm de mercure |
|---|---|
| 1 | 56,5 +/-4,27 p=0,03 |
| 4 | 59 +/-4,33 p=0,02 |

Les levures selon l'invention permettent de restaurer un niveau de perception de la douleur identique à celui observé chez des rats sains. L'effet analgésique induit par les levures est équivalent à celui induit par la morphine.

La figure 26 montre par ailleurs, un effet fortement analgésique du fénofibrate qui augmente d'un facteur 2 le seuil de perception de la douleur chez les rats ayant une hypersensibilité viscérale (70 +/-4,48, p= 0,001).

Ce résultat confirme le rôle du récepteur PPARα dans la modulation de la douleur viscérale.

## Revendications

1. Souche de levure *Saccharomyces cerevisiae* déposée auprès de la Collection Nationale de Cultures de Microorganismes sous le n° CNCM I-3856.

2. Souche de levure *Saccharomyces cerevisiae* var. boulardii déposée auprès de la Collection Nationale de Cultures de Microorganismes sous le n° CNCM I-3799.

3. Composition **caractérisée en ce qu'**elle comprend la souche de levure *Saccharomyces cerevisiae* selon la revendication 1, ou la souche de levure *Saccharomyces cerevisiae* selon la revendication 2 ou l'un de leurs mélanges.

4. Composition selon la revendication 3, **caractérisée en ce que** la souche de levure *Saccharomyces cerevisiae* est sous forme sèche ou fraîche, de préférence sous forme sèche instantanée ou sèche active.

5. Composition selon la revendication 3 ou la revendication 4 comprenant en outre au moins un dérivé de la souche de levure selon la revendication 1 ou la revendication 2 choisi parmi les extraits de levure, les dérivés de parois, les glucanes pariétaux, les mannoprotéines pariétales, les fractions lipidiques de levure, et les fractions d'acides nucléiques de levure (ARN, ADN).

6. Composition selon l'une quelconque des revendications 3 à 5, **caractérisée en ce qu'**elle comprend entre $10^7$ et $6.10^{10}$ UFC, et de préférence entre $10^8$ et $2.10^{10}$ UFC, de souche de levure *Saccharomyces cerevisiae.*

7. Composition selon la revendication 3 ou la revendication 4, **caractérisée en ce qu'**elle comprend entre 1 mg et 10 g, et de préférence entre 1 mg et 1 g, de souche de levure *Saccharomyces cerevisiae.*

8. Composition selon la revendication 5, **caractérisée en ce qu'**elle comprend entre 1 mg et 10 g, et de préférence entre 1 mg et 1 g, de souche de levure *Saccharomyces cerevisiae* et du dérivé de souche de levure.

9. Utilisation de la composition selon l'une quelconque des revendications 3 à 8, pour la préparation d'un complément alimentaire et/ou d'un probiotique et/ou d'un alicament et/ou d'un nutraceutique et/ou d'ingrédients fonctionnels et/ou d'un cosmétique destiné à l'homme et/ou à l'animal.

10. Utilisation de la composition selon l'une quelconque des revendications 3 à 8 pour la préparation de compositions alimentaires destinées à améliorer le confort gastrointestinal et/ou améliorer la flore intestinale.

11. Utilisation de la composition selon l'une quelconque des revendications 3 à 8 pour la préparation d'un médicament destiné au traitement et/ou à la prévention des désordres intestinaux, des troubles fonctionnels intestinaux ou des maladies gastrointestinales.

12. Utilisation de la composition selon l'une quelconque des revendications 3 à 8 pour la préparation d'un médicament destiné au traitement et/ou à la prévention des pathologies ou troubles de l'intestin signalées par un état d'hyperalgésie.

13. Utilisation selon l'une quelconque des revendications 9 à 12, dans laquelle la levure est sous forme sèche ou fraîche, de préférence sous forme sèche instantanée ou sèche active.

14. Utilisation selon l'une quelconque des revendications 9 à 12 de la levure à une dose journalière comprise entre $10^7$ et $6.10^{10}$ UFC, et de préférence entre $10^8$ et $2.10^{10}$ UFC.

15. Utilisation selon l'une quelconque des revendications 9 à 12 de la levure et/ou du dérivé de levure à une dose journalière comprise entre 1 mg et 10 g, et de préférence entre 1 mg et 1 g.

16. Kit comprenant la souche de levure *Saccharomyces cerevisiae* selon la revendication 1, ou la souche de levure *Saccharomyces cerevisiae* selon la revendication 2, ou l'un de leurs mélanges dans une forme adaptée à une administration orale.

17. Kit selon la revendication 16 comprenant en outre au moins un dérivé de la souche de levure selon la revendication 1 ou la revendication 2 choisi parmi les extraits de levure, les dérivés de parois, les glucanes pariétaux, les mannoprotéines pariétales, les fractions lipidiques de levure, et les fractions d'acides nucléiques de levure (ARN, ADN).

**Patentansprüche**

1. Hefestamm *Saccharomyces cerevisiae,* hinterlegt bei der Collection Nationale de Cultures de Microorganismes unter der Nr. CNCM I-3856.

2. Hefestamm *Saccharomyces cerevisiae,* Var. boulardii, hinterlegt bei der Collection Nationale de Cultures de Microorganismes unter der Nr. CNCM I-3799.

3. Zusammensetzung, welche **dadurch gekennzeichnet ist, dass** sie den Hefestamm *Saccharomyces cerevisiae* nach Anspruch 1 umfasst, oder den Hefestamm *Saccharomyces cerevisiae* nach Anspruch 2 oder eine ihrer Mischungen.

4. Zusammensetzung nach Anspruch 3, welche **dadurch gekennzeichnet ist, dass** der Hefestamm *Saccharomyces cerevisiae* trocken oder frisch vorliegt, bevorzugt als trockene Instantform oder aktive Trockenform.

5. Zusammensetzung nach Anspruch 3 oder Anspruch 4, welche ferner wenigstens ein Derivat des Hefestamms nach Anspruch 1 oder Anspruch 2 umfasst, gewählt aus Hefeextrakten, Wandderivaten, parietalen Glucanen, parietalen Mannoproteinen, Hefe-Lipidfraktionen sowie Hefe-Nukleinsäurefraktionen (RNS, DNS).

6. Zusammensetzung nach einem der Ansprüche 3 bis 5, **dadurch gekennzeichnet, dass** sie zwischen $10^7$ und $6.10^{10}$ CFU, und bevorzugt zwischen $10^8$ und $2.10^{10}$ CFU, des Hefestamms *Saccharomyces cerevisiae* umfasst.

7. Zusammensetzung nach Anspruch 3 oder Anspruch 4, **dadurch gekennzeichnet, dass** sie zwischen 1 mg und 10 g, bevorzugt zwischen 1 mg und 1 g des Hefestamms *Saccharomyces cerevisiae* umfasst.

8. Zusammensetzung nach Anspruch 5, **dadurch gekennzeichnet, dass** sie zwischen 1 mg und 10 g, bevorzugt zwischen 1 mg und 1 g des Hefestamms *Saccharomyces cerevisiae* und des Hefestamm-Derivates umfasst.

9. Verwendung der Zusammensetzung nach einem der Ansprüche 3 bis 8 für die Zubereitung eines Nahrungsergänzungsmittels und/oder eines probiotischen Mittels und/oder eines Gesundheitsproduktes und/oder eines Nutrazeutikums und/oder eines funktionellen Bestandteils und/oder eines kosmetischen Mittels für Mensch und/oder Tier.

10. Verwendung der Zusammensetzung nach einem der Ansprüche 3 bis 8 für die Zubereitung von Lebensmittel-Zusammensetzungen, die das gastrointestinale Wohlbefinden und/oder die Darmflora verbessern sollen.

11. Verwendung der Zusammensetzung nach einem der Ansprüche 3 bis 8 für die Zubereitung eines Medikaments für die Behandlung und/oder Prävention von Verdauungsstörungen, funktionalen Darmstörungen oder gastrointestinalen Erkrankungen.

**12.** Verwendung der Zusammensetzung nach einem der Ansprüche 3 bis 8 für die Zubereitung eines Medikaments für die Behandlung und/oder Prävention von Erkrankungen oder Störungen des Darms, die in einer Hyperalgesie Ausdruck finden.

**13.** Verwendung nach einem der Ansprüche 9 bis 12, bei welcher die Hefe in trockener oder frischer Form vorliegt, bevorzugt als trockene Instantform oder aktive Trockenform.

**14.** Verwendung nach einem der Ansprüche 9 bis 12 der Hefe in einer Tagesdosis zwischen $10^7$ und $6.10^{10}$ CFU, bevorzugt zwischen $10^8$ und $2.10^{10}$ CFU.

**15.** Verwendung nach einem der Ansprüche 9 bis 12 der Hefe und/oder des Hefederivats in einer Tagesdosis zwischen 1 mg und 10 g, bevorzugt zwischen 1 mg und 1 g.

**16.** Kit, welches den Hefestamm *Saccharomyces cerevisiae* nach Anspruch 1 umfasst, oder den Hefestamm *Saccharomyces cerevisiae* nach Anspruch 2, oder eine ihrer Mischungen, in einer für eine orale Darreichung geeigneten Form.

**17.** Kit nach Anspruch 16, welches ferner wenigstens ein Derivat des Hefestamms nach Anspruch 1 oder Anspruch 2 umfasst, gewählt aus Hefeextrakten, Wandderivaten, parietalen Glucanen, parietalen Mannoproteinen, Hefe-Lipidfraktionen sowie Hefe-Nukleinsäurefraktionen (RNS, DNS).


**Claims**

**1.** *Saccharomyces cerevisiae* yeast strain deposited with the Collection Nationale de Cultures de Microorganismes [French National Collection of Microorganism Cultures] under No. CNCM 1-3856.

**2.** *Saccharomyces cerevisiae* var. boulardii yeast strain deposited with the Collection Nationale de Cultures de Microorganismes under No. CNCM 1-3799.

**3.** Composition **characterized in that** it comprises the *Saccharomyces cerevisiae* yeast strain according to claim 1, or the *Saccharomyces cerevisiae* yeast strain according to claim 2 or a mixture thereof.

**4.** Composition according to claim 3, **characterized in that** the *Saccharomyces cerevisiae* yeast strain is in dry or fresh form, preferably in instant dry or active dry form.

**5.** Composition according to claim 3 or claim 4, further comprising at least one derivative of the yeast strain according to claim 1 or claim 2 chosen from yeast extracts, wall derivatives, wall glucans, wall mannoproteins, yeast lipid fractions, and yeast nucleic acid fractions (RNA, DNA).

**6.** Composition according to any one of claims 3 to 5, **characterized in that** it comprises between $10^7$ and $6 \times 10^{10}$ CFU, and preferably between $10^8$ and $2 \times 10^{10}$ CFU, of *Saccharomyces cerevisiae* yeast strain.

**7.** Composition according to claim 3 or claim 4, **characterized in that** it comprises between 1 mg and 10 g, and preferably between 1 mg and 1 g, of *Saccharomyces cerevisiae* yeast strain.

**8.** Composition according to claim 5, **characterized in that** it comprises between 1 mg and 10 g, and preferably between 1 mg and 1 g, of *Saccharomyces cerevisiae* yeast strain and of the yeast strain derivative.

**9.** Use of the composition according to any one of claims 3 to 8, for preparing a food supplement and/or a probiotic and/or a functional food and/or a nutraceutical and/or functional ingredients and/or a cosmetic intended for human beings and/or for animals.

**10.** Use of the composition according to any one of claims 3 to 8, for preparing food compositions intended to improve gastrointestinal comfort and/or to improve intestinal flora.

**11.** Use of the composition according to any one of claims 3 to 8, for preparing a medicament intended for the treatment and/or prevention of intestinal disorders, of functional intestinal ailments or of gastrointestinal diseases.

12. Use of the composition according to any one of claims 3 to 8, for preparing a medicament intended for the treatment and/or prevention of pathological conditions or ailments of the intestine indicated by a hyperalgesic state.

13. Use according to any one of claims 9 to 12, in which the yeast is in dry or fresh form, preferably in instant dry or active dry form.

14. Use according to any one of claims 9 to 12 of the yeast at a daily dose of between $10^7$ and $6 \times 10^{10}$ CFU, and preferably between $10^8$ and $2 \times 10^{10}$ CFU.

15. Use according to any one of claims 9 to 12 of the yeast and/or of the yeast derivative at a daily dose of between 1 mg and 10 g, and preferably between 1 mg and 1 g.

16. Kit comprising the *Saccharomyces cerevisiae* yeast strain according to claim 1, or the *Saccharomyces cerevisiae* yeast strain according to claim 2, or a mixture thereof, in a form suitable for oral administration.

17. Kit according to claim 16, also comprising at least one derivative of the yeast strain according to claim 1 or claim 2, chosen from yeast extracts, wall derivatives, wall glucans, wall mannoproteins, yeast lipid fractions, and yeast nucleic acid fractions (RNA, DNA).

Fig. 1

Fig. 2

**Fig. 3**

**Fig. 4**

**Pré-incubation**

**Fig. 5**

**Co-incubation**

**Fig. 6**

Fig. 7

Fig. 8

Fig. 9

Fig. 10

**Fig. 11**

**Fig. 12**

Fig. 13

Fig. 14

Fig. 15

Fig. 16

Fig. 17

Test ELISA: mannoprotéines de levures

Fig. 18

**Fig. 19A**

**Fig. 19B**

Fig. 20A

Fig. 20B

**Fig. 21A**

**Fig. 21B**

Fig. 22A

Fig. 22B

Fig. 23

**Fig. 24**

**Fig. 25**

**Fig. 26**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• WO 2006021965 A

**Littérature non-brevet citée dans la description**

• **M. DE VRESE ; P.R. MARTEAU.** Probiotics and Prebiotics: Effects on Diarrhea. *J. Nutr.,* 2007, vol. 137 (3), 8035-811S

• Probiotics in Food Safety and Human Health. CRC Taylor & Francis, 2006